# EUROPÄISCHE PATENTSCHRIFT

(11) **EP 2 628 762 B1**
(45) Veröffentlichungstag und Bekanntmachung des Hinweises auf die Patenterteilung: **10.04.2019**
(21) Anmeldenummer: 13155900.7
(22) Anmeldetag: 20.02.2013
(51) Int. Cl.: C08G 75/16, C09K 21/14, C07C 61/00, C08F 10/00, C08L 7/00, C08L 23/00

(54) **VERFAHREN ZUR HERSTELLUNG VON POLYPHENOLDISULFIDEN**
METHOD FOR MANUFACTURING POLYPHENOL DISULFIDES
PROCÉDÉ DESTINÉ À LA FABRICATION DE POLYPHÉNOLES DISULFURÉS

(30) Priorität: 20.02.2012 EP 12156228
(43) Veröffentlichungstag der Anmeldung: 21.08.2013
(73) Patentinhaber: BASF SE, 67056 Ludwigshafen am Rhein (DE)
(72) Erfinder: Fleckenstein, Christoph, 63579 Freigericht (DE); Denecke, Hartmut, 67071 Ludwigshafen (DE); Fuchs, Sabine, 68167 Mannheim (DE); Müller, Matthias, 64319 Pfungstadt (DE); Döring, Manfred, 64289 Darmstadt (DE); Cisielski, Michael, 06217 Merseburg (DE); Wagner, Jochen, 67112 Mutterstadt (DE); Deglmann, Peter, 68163 Mannheim (DE); Nalawade, Sameer, 68159 Mannheim (DE); Gutmann, Peter, 76199 Karlsruhe (DE); Hahn, Klaus, 67281 Kirchheim (DE); Massonne, Klemens, 67098 Bad Dürkheim (DE); Kleinke, Andreas, 69115 Heidelberg (DE); Krämer, Roland Helmut, 68165 Mannheim (DE); Mailahn, Elmar, 67547 Worms (DE)

(56) Entgegenhaltungen:
- GB-A- 1 129 546
- GB-A- 1 268 971
- US-A- 1 756 817
- US-A- 2 422 156
- US-A- 2 445 737
- US-A- 3 284 544
- US-A- 3 812 192
- US-A- 3 968 062
- US-A1- 2007 093 613
- BREZHNEVA ET AL: "REACTION OF p-tert-BUTYLPHENOL AND 2-METHYL-4-tert-BUTYLPHENOL WITH SULFUR MONO- AND DICHLORIDE", JOURNAL OF ORGANIC CHEMISTRY OF THE USSR = ZHURNAL ORGANICHESKOI KHIMII, M A I K NAUKA - INTERPERIODICA, RU, Bd. 17, Nr. 9, 1. Januar 1981 (1981-01-01) , Seiten 1723-1726, XP008163643, ISSN: 0022-3271
- TH. ZINCKE ET AL: "Über Schwefelderivate des p-Kresols", BERICHTE DER DEUTSCHEN CHEMISCHEN GESELLSCHAFT, Bd. 44, Nr. 1, 1. Januar 1911 (1911-01-01) , Seiten 413-424, XP055035022, ISSN: 0365-9496, DOI: 10.1002/cber.19110440164

## Beschreibung

Die Erfindung betrifft ein Verfahren zur Herstellung von Polyphenoldisulfiden. Weiter betrifft die Erfindung Polyphenoldisulfide, erhältlich durch das Verfahren, ein Flammschutzsystem, enthaltend das Polyphenoldisulfid und eine halogenfreie organische Phosphorverbindung, sowie eine Polymerzusammensetzung, enthaltend das Flammschutzsystem.

Die Ausrüstung von Polymeren, insbesondere Schaumstoffen, mit Flammschutzmitteln ist für eine Vielzahl von Anwendungen von Bedeutung, beispielsweise für Polystyrol-Partikelschaumstoffe aus expandierbarem Polystyrol (EPS) oder Polystyrol-Extrusionsschaumstoffplatten (XPS) zum Isolieren von Gebäuden.

Derzeit werden als Flammschutzmittel in Kunststoffen hauptsächlich polyhalogenierte Kohlenwasserstoffe, gegebenenfalls zusammen mit geeigneten Synergisten, eingesetzt. Ein typischer Vertreter dieser klassischen Flammschutzmittel ist Hexabromcyclododecan. Aufgrund von Bioakkumulation sowie Persistenz einiger polyhalogenierter Kohlenwasserstoffe ist es ein großes Bestreben in der Kunststoffindustrie, halogenhaltige Flammschutzmittel durch halogenfreie zu ersetzen.

Eine vielversprechende Alternative zu halogenhaltigen Systemen bieten halogenfreie phosphorhaltige Flammschutzmittel in Kombination mit halogenfreien schwefelhaltigen Synergisten.

Der Einsatz von Polyphenoldisulfiden als Synergisten zu halogenfreien phosphorhaltigen Flammschutzmitteln wird bereits in AT 508 304 und WO 2011/000019 sowie in der nicht vorveröffentlichten internationalen Patentanmeldung PCT/EP2011/073958 beschrieben.

Poly-(*tert*.-butylphenoldisulfid) und Poly-(*tert*.-pentylphenoldisulfid) sind wichtige Vertreter dieser Verbindungsklasse und können zum Beispiel als VULTAC TB7 bzw. VULTAC 2 und 3 kommerziell bezogen werden. Gemäß US 3,968,062 wird davon ausgegangen, dass es sich hierbei um komplexe Mischungen aus Polysulfiden handelt.

Nach US 3,968,062 wird Poly-(*tert*.-butylphenoldisulfid) durch Erhitzen von para-*tert.-*Butylphenol mit Schwefelmonochlorid unter Verwendung von Trichlorethylen als Lösungsmittel hergestellt, wobei die Temperatur des Heizmantels zwischen 115 und 155 °C liegt. Der Einsatz eines niedrig siedenden Lösungsmittels wie Trichlorethylen empfiehlt sich gemäß oben genannter US-Patentanmeldung, um sublimiertes para-*tert*.-Butylphenol in das Reaktionsgefäß zurückzuführen.

US 3,812,192 beschreibt ein Verfahren zur Herstellung von Polythiobisphenolen aus Phenolen und Dischwefeldichlorid.

US 2007/093613 offenbart die Herstellung dimerer und polymerer Alkylphenolpolysulfide auf Basis von Paracumylphenol zur Anwendung in vulkanisierbarem Kautschuk.

In US 3,284,544 sind selbstlöschende Alkenylaromatische Harze enthaltend ein Halogenid als Flammschutzmittel gezeigt, wobei ein organisches Disulfid zur Verbesserung der Flammschutzeigenschaften des Halogenids zugesetzt wird.

US 2,422,156 beschreibt die Verwendung von bestimmten Phenolsulfiden in der Herstellung synthetischer Kautschukprodukte aus 1,3-butadienpolymerisaten.

In US 2,445,737 sind synthetische Kautschukzusammensetzungen offenbart, denen Harze zugesetzt werden, welche aus substituierten Phenolen, Schwefeldichlorid und aldehyden hergestellt sind.

GB 1 268 971 offenbart vulkanisierbare Kautschukzusammensetzungen, die mit dem Produkt der Reaktion eines Phenols mit Schwefelmonochlorid in Abwesenheit eines Formaldehyddonors umgesetzt werden.

In US 1,756,817 wird ein Verfahren beschrieben, durch das ein kommerziell nutzbares Harz durch Reaktion eines Schwefelchlorids mit Phenolen hergestellt wird.

GB 1 129 546 beschreibt ein Verfahren, bei dem Schwierigkeiten beim Bleichen von Rosin- und Kiefernölverbindungen dadurch umgangen werden, dass diese Stoffe bei erhöhten Temperaturen mit Polyphenoldisulphiden in Verbindung gebracht werden.

Brezhneva et al. beschreiben im "Journal of Organic Chemistry of the USSR" (Bd. 17, Nr. 9, 1. Januar 1981, Seiten 1723 - 1726) Reaktionen von p-tert-Butylphenol und 2-Methyl-4-tert-butylphenol mit Schwefelmono- und dichlorid.

In der Zeitschrift "Berichte der Deutschen Chemischen Gesellschaft" (Bd. 44 Nr.1, 1. Januar 1911, Seiten 413 - 424) berichten Zincke et al. über Schwefelderivate des p-Kresols.

Obwohl die Polyphenoldisulfide des Standes der Technik effiziente Synergisten zu halogenfreien phosphorhaltigen Flammschutzmitteln darstellen, besteht noch Raum für Verbesserungen, insbesondere was deren Herstellung sowie deren anwendungstechnische Eigenschaften betrifft.

Ein Nachteil kommerziell erhältlicher Polysulfidmischungen ist deren unbefriedigende thermische Stabilität für die Einarbeitung in thermoplastische Polymere, z. B. Polystyrol, sowie der darin enthaltene Anteil an niedermolekularen, leichtflüchtigen Komponenten. Damit verbunden ist auch eine während ihrer Anwendung in Polymeren bei erhöhter Temperatur teilweise auftretende Geruchsentwicklung, z. B. bei der Extrusion oder beim Zuschnitt von Schaumstoffblöcken.

Die Aufgabe der Erfindung liegt in der Bereitstellung eines Verfahrens zur Herstellung von Polyphenoldisulfiden, die eine gute thermische Stabilität sowie einen geringen Anteil an niedermolekularen, leichtflüchtigen Komponenten zeigen und während deren Anwendung in Polymeren bei erhöhter Temperatur kein Geruch entsteht.

Es wurde gefunden, dass die Umsetzung von Phenolen mit S₂Cl₂ in einem Lösungsmittel, das im Laufe der Umsetzung entstehende HCl komplexiert und/oder neutralisiert oder in dem HCl unlöslich ist, zur Bildung von Polyphenoldisulfiden führt, die im Vergleich zu Polyphenoldisulfiden des Standes der Technik einen höheren Anteil an dimeren und polymeren Phenoldisulfiden aufweisen und sich durch verbesserte anwendungstechnische Eigenschaften auszeichnen.

Gelöst wird die Aufgabe daher durch ein Verfahren zur Herstellung eines Polyphenoldisulfids der Formel (I) 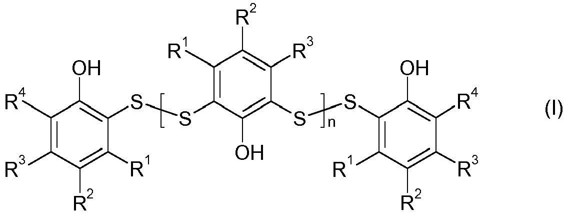 durch Umsetzung von einem oder mehreren Phenolen der Formel (II) 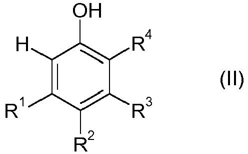 mit S₂Cl₂ (III) in einem Lösungsmittel L,
wobei das Lösungsmittel L die Eigenschaft besitzt, dass
a) es mindestens 80 mol-% der im Laufe der Umsetzung entstehenden HCl komplexiert und/oder neutralisiert, wobei das Lösungsmittel L eine oder mehrere Lewis- und/oder Brønsted-basische Lösungsmittelkomponenten enthält, welche HCl komplexieren und/oder neutralisieren, wobei die einen oder mehreren Lewis- und/oder Brønsted-basischen Lösungsmittelkomponenten Nitrile mit 2-8 C-Atomen pro Cyanogruppe, Carbonsäureamide mit 2 bis 8 C-Atomen pro Carbonsäureamidgruppe, Harnstoffe mit 2 bis 8 C-Atomen pro Harnstoffgruppe, Thioharnstoffe mit 2 bis 8 C-Atomen pro Thioharnstoffgruppe, Sulfoxide mit 2 bis 8 C-Atomen pro Sulfoxygruppe, Amine mit 4 bis 20 C-Atomen pro Aminogruppe sind; oder
b) mindestens 80 mol-% der im Laufe der Umsetzung entstehenden HCl in dem Lösungsmittel L unlöslich ist;
und
wobei die Symbole R¹, R², R³, R⁴ und n folgende Bedeutungen haben:
- R¹, R², R³, R⁴: sind gleich oder verschieden C₁-C₁₈-Alkyl, C₂-C₁₈-Alkenyl, C₂-C₁₈-Alkinyl, C₆-C₁₂-Aryl, C₃-C₁₀-Cycloalkyl, C₆-C₁₂-Aryl-C₁-C₁₈-Alkyl, eine Heteroarylgruppe, die ein oder mehrere Heteroatome aus der Gruppe N, O und S enthält, O-(C₁-C₁₈)-Alkyl, O-(C₁-C₁₈)-Alkenyl, O-(C₂-C₁₈)-Alkinyl, O-(C₆-C₁₂)-Aryl, O-(C₃-C₁₀)-Cycloalkyl, (C₆-C₁₂)-Aryl-(C₁-C₁₈)-Alkyl-O, S-(C₁-C₁₈)-Alkyl, S-(C₂-C₁₈)-Alkenyl, S-(C₂-C₁₈)-Alkinyl, S-(C₆-C₁₂)-Aryl, S-(C₃-C₁₀)-Cycloalkyl, (C₆-C₁₂)-Aryl-(C₁-C₁₈)-Alkyl-S, OH, F, Cl, Br oder H;
- n: ist eine ganze Zahl von 1 bis 1000, vorzugsweise eine ganze Zahl von 3 bis 1000;
mit der Maßgabe, dass R⁴ in mindestens einem der einen oder mehreren Phenole der Formel (II) H ist.

Erfindungsgemäß hergestelltes Polyphenoldisulfid weist im Vergleich zu Polyphenoldisulfiden des Standes der Technik einen höheren Anteil an dimeren und polymeren Phenoldisulfiden, einen geringeren Anteil an Monosulfideinheiten, einen geringeren Anteil an Polysulfideinheiten sowie einen geringeren Gehalt an elementarem Schwefel auf. Die nach dem erfindungsgemäßen Verfahren hergestellten Polyphenoldisulfide zeichnen sich durch eine zufriedenstellende thermische Stabilität aus. Damit verbunden ist auch eine während ihrer Anwendung in Polymeren bei erhöhter Temperatur kaum oder gar nicht auftretende Geruchsentwicklung, z. B. bei der Extrusion oder beim Zuschnitt von Schaumstoffblöcken. Ferner weist erfindungsgemäß hergestelltes Polyphenoldisulfid nur einen geringen Gehalt an kovalent gebundenem Chlor, z. B. in Form von chlorierten Aromaten, auf. Darüber hinaus wird das erfindungsgemäße Verfahren bevorzugt bei vergleichsweise geringen Temperaturen durchgeführt, was aus ökonomischer und ökologischer Sicht vorteilhaft ist. Unter anderem wird dadurch auch der Sublimation von nicht reagiertem Phenol entgegengewirkt.

Im Rahmen der Erfindung umfasst der Begriff "Polyphenoldisulfid der Formel (I)" dimere Phenoldisulfide und polymere Phenoldisulfide.

Unter einem erfindungsgemäßen dimeren Phenoldisulfid oder einem erfindungsgemäßen Diphenoldisulfid versteht man eine Verbindung der Formel (I), wobei n 0 ist.

Beispiele für erfindungsgemäße dimere Phenoldisulfide oder erfindungsgemäße Diphenoldisulfide sind die in den Beispielen aufgeführten Verbindungen Di-(*tert*.-butylphenol)disulfid und Di-(*tert*.-pentylphenol)disulfid.

Unter einem erfindungsgemäßen polymeren Phenoldisulfid versteht man eine Verbindung der Formel (I), wobei n nicht 0 ist und wobei höchstens 50%, vorzugsweise ≤ 35%, besonders bevorzugt ≤ 20% der in der Formel (I) enthaltenen (n+1) Disulfidbrücken durch eine Mono- oder Oligosulfidbrücke ersetzt sind. Im Rahmen der Erfindung versteht man unter einer Oligosulfidbrücke eine lineare Kette, bestehend aus 3 oder mehr, insbesondere 3 bis 8 Schwefelatomen.

Beispiele für erfindungsgemäße polymere Phenoldisulfide sind die in den Beispielen aufgeführten Verbindungen Poly-(*tert*.-butylphenoldisulfid) und Poly-(*tert*.-pentylphenoldisulfid).

Bevorzugt haben die Symbole in der Formel (I) folgende Bedeutungen:
- R²: ist bevorzugt gleich oder verschieden C₁-C₁₈-Alkyl, C₂-C₁₈-Alkenyl, C₂-C₁₈-Alkinyl, C₆-C₁₂-Aryl, C₃-C₁₀-Cycloalkyl, O-(C₁-C₁₈)-Alkyl, O-(C₁-C₁₈)-Alkenyl, O-(C₂-C₁₈)-Alkinyl, O-(C₆-C₁₂)-Aryl oder O-(C₃-C₁₀)-Cycloalkyl.
- R¹, R³, R⁴: sind bevorzugt gleich oder verschieden H, C₁-C₁₈-Alkyl, C₂-C₁₈-Alkenyl, C₂-C₁₈-Alkinyl, C₆-C₁₂-Aryl, C₃-C₁₀-Cycloalkyl, O-(C₁-C₁₈)-Alkyl, O-(C₂-C₁₈)-Alkenyl, O-(C₂-C₁₈)-Alkinyl, O-(C₆-C₁₂)-Aryl oder O-(C₃-C₁₀)-Cycloalkyl.
- n: ist bevorzugt eine ganze Zahl von 1 bis 500.

Bevorzugt sind Polyphenoldisulfide der Formel (I), in denen alle Symbole die bevorzugten Bedeutungen haben.

Ebenfalls bevorzugt sind Polyphenoldisulfide der Formel (I), in denen n eine ganze Zahl von 3 bis 1000 ist.

Besonders bevorzugt haben die Symbole in der Formel (I) folgende Bedeutungen:
- R²: ist besonders bevorzugt gleich oder verschieden C₁-C₁₆-Alkyl oder C₆-C₁₂-Aryl.
- R¹, R³, R⁴: sind besonders bevorzugt gleich oder verschieden H, C₁-C₁₈-Alkyl, C₃-C₁₀-Cycloalkyl oder C₆-C₁₂-Aryl.
- n: ist besonders bevorzugt eine ganze Zahl von 1 bis 250.

Besonders bevorzugt sind Polyphenoldisulfide der Formel (I), in denen alle Symbole die besonders bevorzugten Bedeutungen haben.

Ganz besonders bevorzugt haben die Symbole in der Formel (I) folgende Bedeutungen:
- R²: ist ganz besonders bevorzugt gleich oder verschieden C₁-C₁₀-Alkyl oder C₆-C₁₂-Aryl.
- R¹, R³, R⁴: sind ganz besonders bevorzugt H.
- n: ist ganz besonders bevorzugt eine ganze Zahl von 1 bis 150.

Ganz besonders bevorzugt sind Polyphenoldisulfide der Formel (I), in denen alle Symbole die ganz besonders bevorzugten Bedeutungen haben.

Ebenfalls ganz besonders bevorzugt sind Polyphenoldisulfide der Formel (I), in denen n eine ganze Zahl von 3 bis 100 ist.

Weiterhin ganz besonders bevorzugt sind die in den Beispielen aufgeführten Verbindungen der Formel (I), Poly-(*tert*.-butylphenoldisulfid) und Poly-(*tert*.-pentylphenoldisulfid).

Ganz besonders bevorzugt ist auch die Verbindung Poly-(para-phenylphenoldisulfid):

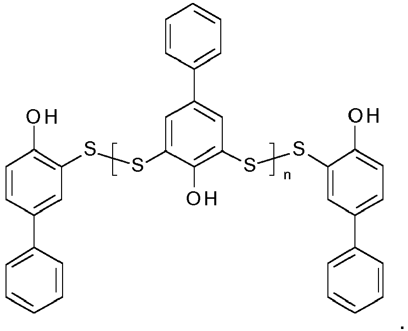

Insbesondere bevorzugt haben die Symbole in der Formel (I) folgende Bedeutungen:
- R²: ist insbesondere bevorzugt gleich oder verschieden t-C₄H₉ oder t-C₅H₁₁.
- R¹, R³, R⁴: sind insbesondere bevorzugt H.
- n: ist insbesondere bevorzugt eine ganze Zahl von 3 bis 100.

Insbesondere bevorzugt sind Polyphenoldisulfide der Formel (I), in denen alle Symbole die insbesondere bevorzugten Bedeutungen haben.

Weiterhin insbesondere bevorzugt sind die in den Beispielen aufgeführten Verbindungen der Formel (I), Poly-(*tert*.-butylphenoldisulfid) und Poly-(*tert*.-pentylphenoldisulfid).

Ein weiterer Gegenstand der Erfindung ist ein Verfahren zur Herstellung eines Polyphenoldisulfids der Formel (I) 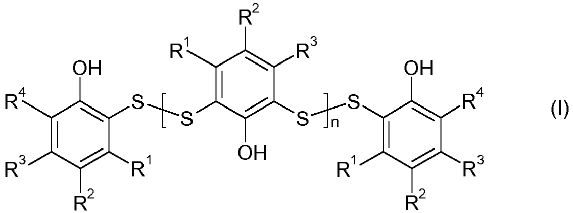 durch Umsetzung von einem oder mehreren Phenolen der Formel (II) 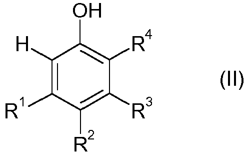 mit S₂Cl₂ (III) in einem Lösungsmittel L,
wobei das Lösungsmittel L die Eigenschaft besitzt, dass es mindestens 80 mol-% der im Laufe der Umsetzung entstehenden HCl komplexiert und/oder neutralisiert, wobei das Lösungsmittel L eine oder mehrere Lewis- und/oder Brønsted-basische Lösungsmittelkomponenten enthält, welche HCl komplexieren und/oder neutralisieren, wobei die einen oder mehreren Lewis- und/oder Brønsted-basischen Lösungsmittelkomponenten Nitrile mit 2-8 C-Atomen pro Cyanogruppe, Carbonsäureamide mit 2 bis 8 C-Atomen pro Carbonsäureamidgruppe, Harnstoffe mit 2 bis 8 C-Atomen pro Harnstoffgruppe, Thioharnstoffe mit 2 bis 8 C-Atomen pro Thioharnstoffgruppe, Sulfoxide mit 2 bis 8 C-Atomen pro Sulfoxygruppe, Amine mit 4 bis 20 C-Atomen pro Aminogruppe sind;
und
wobei die Symbole R¹, R², R³, R⁴ und n folgende Bedeutungen haben:
- R²: ist gleich oder verschieden C₁-C₁₀-Alkyl oder C₆-C₁₂-Aryl;
- R¹, R³, R⁴: sind H;

- n: ist eine ganze Zahl von 0 bis 150.

Dabei bevorzugt sind die in den Beispielen aufgeführten Verbindungen der Formel (I), Di-(*tert*.-butylphenol)disulfid, Di-(*tert*.-pentylphenol)disulfid, Poly-(*tert*.-butylphenoldisulfid) und Poly-(*tert*.-pentylphenoldisulfid), sowie die Verbindungen Di-(para-phenylphenol)disulfid und Poly-(para-phenylphenoldisulfid):

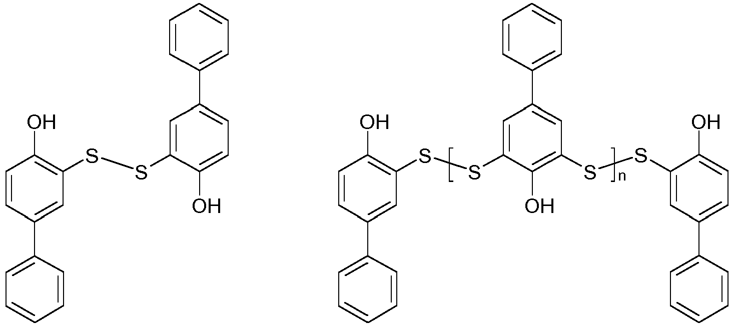

Ein weiteres Beispiel für eine Verbindung der Formel (I) ist Di-(ortho-phenylphenol)disulfid:

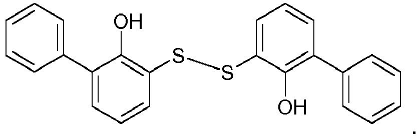

In den erfindungsgemäßen Verfahren werden ein oder mehrere, vorzugsweise ein bis drei, besonders bevorzugt ein (1) Phenol der Formel (II) eingesetzt.

Bevorzugt sind Phenole der Formel (II), die zur Bildung der bevorzugten Polyphenoldisulfide der Formel (I) führen.

Besonders bevorzugt sind Phenole der Formel (II), die zur Bildung der besonders bevorzugten Polyphenoldisulfide der Formel (I) führen.

Ganz besonders bevorzugt sind Phenole der Formel (II), die zur Bildung der ganz besonders bevorzugten Polyphenoldisulfide der Formel (I) führen. Beispiele für ganz besonders bevorzugte Phenole der Formel (II) sind para-*tert*.-Butylphenol, para*-tert.-*Pentylphenol und para-Phenylphenol.

Insbesondere bevorzugt sind Phenole der Formel (II), die zur Bildung der insbesondere bevorzugten Polyphenoldisulfide der Formel (I) führen. Hierbei handelt es sich um die Verbindungen para-*tert*.-Butylphenol und para-*tert*.-Pentylphenol.

Ein weiteres Beispiel für ein Phenol der Formel (II) ist ortho-Phenylphenol.

Die in dem erfindungsgemäßen Verfahren eingesetzten einen oder mehreren Phenole der Formel (II) sind kommerziell erhältlich oder können in Anlehnung an literaturbekannte Methoden synthetisiert werden.

Zum Beispiel können die insbesondere bevorzugten Verbindungen der Formel (II), p-*tert*.-Butylphenol und p-*tert*.-Pentylphenol, durch Friedel-Crafts-Alkylierung von Phenol mit Isobutylen bzw. Isopentylen erhalten werden.

S₂Cl₂ (III) - Schwefelmonochlorid - ist kommerziell erhältlich.

Üblicherweise werden S₂Cl₂ (III) und das Phenol der Formel (II) in einem Molverhältnis von 1:1 bis 1:4, bevorzugt 1:1 bis 1:3, besonders bevorzugt 1:1 bis 1:2,2 miteinander umgesetzt.

Durch Variation des Molverhältnisses von S₂Cl₂ (III) zu dem Phenol der Formel (II) lässt sich der Oligomerisierungsgrad der Verbindung der Formel (I) beeinflussen.

Erfindungsgemäß erfolgt die Umsetzung in einem Lösungsmittel L.

Das Lösungsmittel L enthält eine oder mehrere, vorzugsweise eine bis drei, besonders bevorzugt eine oder zwei Lösungsmittelkomponenten.

Üblicherweise werden 100 bis 3000 ml, vorzugsweise 200 bis 2000 ml, besonders bevorzugt 300 bis 1000 ml Lösungsmittel L pro mol Phenol der Formel (II) eingesetzt.

In einer bevorzugten Ausführungsform (a) besitzt das Lösungsmittel L die Eigenschaft, dass es mindestens 80 mol-%, vorzugsweise ≥ 90 mol-%, besonders bevorzugt ≥ 95 mol-%, ganz besonders bevorzugt ≥ 98 mol-%, insbesondere bevorzugt ≥ 99 mol-% der im Laufe der Umsetzung entstehenden HCl komplexiert und/oder neutralisiert.

In einer weiteren Ausführungsform (b) besitzt das Lösungsmittel L die Eigenschaft, dass mindestens 80 mol-%, vorzugsweise ≥ 90 mol-%, besonders bevorzugt ≥ 95 mol-%, ganz besonders bevorzugt ≥ 98 mol-%, insbesondere bevorzugt ≥ 99 mol-% der im Laufe der Umsetzung entstehenden HCl in dem Lösungsmittel L unlöslich ist.

Gemäß der Ausführungsform (a) versteht man unter einem Lösungsmittel, das HCl komplexiert und/oder neutralisiert, ein Lösungsmittel, welches als Lewis-Base gegenüber dem H-Atom von HCl und/oder als Brønsted-Base gegenüber dem Proton von HCl fungiert. Ausgehend von dem H-Atom bzw. Proton von HCl können dabei eine oder mehrere koordinative Bindungen bzw. Wasserstoffbrückenbindungen und/oder eine kovalente Bindung zu einem oder mehreren basischen Strukturelementen des Lösungsmittels gemäß der Ausführungsform (a) geknüpft werden, wobei HCl-Addukte des Lösungsmittels entstehen.

In der Ausführungsform (a) enthält das Lösungsmittel L mindestens die Menge an einer oder mehreren Lewis- und/oder Brønsted-basischen Lösungsmittelkomponenten, die stöchiometrisch erforderlich ist, um mindestens 80 mol-%, vorzugsweise ≥ 90 mol-%, besonders bevorzugt ≥ 95 mol-%, ganz besonders bevorzugt ≥ 98 mol-%, insbesondere bevorzugt ≥ 99 mol-% der im Laufe der Umsetzung entstehenden HCl zu komplexieren und/oder zu neutralisieren.

Überraschenderweise wurde für die Umsetzung von S₂Cl₂ eine ausgeprägte Abhängigkeit von dem pK_{b}-Wert der Lösungsmittel gemäß der Ausführungsform (a) festgestellt. Die höchste Selektivität hinsichtlich der Bildung der gewünschten dimeren und polymeren Phenoldisulfide wurde bei Verwendung von moderat bis relativ schwach basischen Lösungsmitteln gefunden wie beispielsweise N,N,N',N'-Tetramethylharnstoff, der einen pK_{b}-Wert von 12 besitzt. Die Selektivität hinsichtlich der Bildung von dimeren und polymeren Phenoldisulfiden nimmt mit sinkendem pK_{b}-Wert ab.

Daher besitzen die erfindungsgemäßen Lösungsmittel in einer bevorzugten Ausführungsform eine moderate bis relativ geringe Basenstärke mit pK_{b}-Werten im Bereich von 6 bis 18.

In einer besonders bevorzugten Ausführungsform besitzen die erfindungsgemäßen Lösungsmittel eine moderate Basenstärke mit pK_{b}-Werten im Bereich von 10 bis 16.

In einer bevorzugten Ausführungsform enthält das erfindungsgemäße Lösungsmittel L eine oder mehrere, vorzugsweise eine bis drei, besonders bevorzugt eine oder zwei Lösungsmittelkomponenten, von denen mindestens eine Komponente auf Grund ihrer Lewis- bzw. Brønsted-basischen Eigenschaften HCl-Addukte bilden kann.

In einer besonders bevorzugten Ausführungsform enthält das Lösungsmittel L mindestens die stöchiometrisch erforderliche Menge einer oder mehrerer Lewis- und/oder Brønsted-basischer Lösungsmittelkomponenten, welche zur vollständigen Bindung der HCl als HCl-Addukt erforderlich ist.

In einer besonderen Ausführungsform enthält das Lösungsmittel L eine oder mehrere Lewis- und/oder Brønsted-basische Lösungsmittelkomponenten, welche HCl komplexieren und/oder neutralisieren.

Als Lewis- und/oder Brønsted-basische Lösungsmittelkomponenten eignen sich beispielsweise Nitrile mit 2 bis 8 C-Atomen pro Cyanogruppe, Carbonsäureamide mit 2 bis 8 C-Atomen pro Carbonsäureamidgruppe, Harnstoffe mit 2 bis 8 C-Atomen pro Harnstoffgruppe, Thioharnstoffe mit 2 bis 8 C-Atomen pro Thioharnstoffgruppe, Sulfoxide mit 2 bis 8 C-Atomen pro Sulfoxygruppe, Amine mit 4 bis 20 C-Atomen pro Aminogruppe.

Beispiele für erfindungsgemäße Nitrile sind Acetonitril, Propionitril, Butyronitril, Benzonitril.

Beispiele für erfindungsgemäße Carbonsäureamide sind N,N-Dimethylformamid (DMF), N,N-Diethylformamid, N,N-Dimethylacetamid (DMAC), N,N-Dimethylpropionamid, N-Methyl-2-pyrolidinon (NMP).

Beispiele für Harnstoffe sind N,N,N',N'-Tetramethylharnstoff, 1,3-Dimethyl-2-imidazolidinon (DMI), 1,3-Dimethyl-3,4,5,6-tetrahydro-2(1H)-pyrimidinon (DMPU).

Ein Beispiel für einen erfindungsgemäßen Thioharnstoff ist N,N,N',N'-Tetramethylthioharnstoff.

Beispiele für erfindungsgemäße Sulfoxide sind Dimethylsulfoxid und Diethylsulfoxid.

Beispiele für erfindungsgemäße Amine sind sind Triethylamin, Diethylamin, N-Ethyldiisopropylamin, Pyridin, Lutidin, Collidin.

Bevorzugte Lewis- und/oder Brønsted-basische Lösungsmittelkomponenten sind C₂-C₈-Nitrile, C₂-C₈-Carbonsäureamide, C₂-C₈-Harnstoffe, C₂-C₈-Thioharnstoffe, C₂-C₈-Sulfoxide, C₄-C₂₀-Amine.

Besonders bevorzugte Lewis- und/oder Brønsted-basische Lösungsmittelkomponenten sind, C₂-C₈-Carbonsäureamide, C₂-C₈-Harnstoffe, C₂-C₈-Sulfoxide.

Ganz besonders bevorzugte Lewis- und/oder Brønsted-basische Lösungsmittelkomponenten sind N,N-Dimethylformamid, N,N,N',N'-Tetramethylharnstoff, 1,3-Dimethyl-2-imidazolidinon, 1,3-Dimethyl-3,4,5,6-tetrahydro-2(1H)-pyrimidinon.

Insbesondere bevorzugte Lewis- und/oder Brønsted-basische Lösungsmittelkomponenten sind DMF und N,N,N',N'-Tetramethylharnstoff.

Die eine oder mehreren, vorzugsweise eine oder zwei, besonders bevorzugt eine (1) Lewis- und/oder Brønsted-basische Lösungsmittelkomponente kann zusammen mit einer oder mehreren, vorzugsweise einer oder zwei, besonders bevorzugt eine (1) zusätzliche Lösungsmittelkomponente L_{Z} eingesetzt werden.

Als zusätzliche Lösungsmittelkomponenten L_{Z} eignen sich beispielsweise Benzol, Alkylbenzole, Alkane, Cycloalkane, halogenierte Kohlenwasserstoffe.

Bevorzugte zusätzliche Lösungsmittelkomponenten L_{Z} sind Alkylbenzole.

Besonders bevorzugte zusätzliche Lösungsmittelkomponenten L_{Z} sind Toluol, Xylole, z. B. ein Xylol-Isomerengemisch, und Ethylbenzol.

In der Ausführungsform (b) besitzt das Lösungsmittel L bevorzugt die Eigenschaft, dass HCl in dem Lösungsmittel L bei einer Temperatur von 20 °C und einem Druck von 1013 mbar eine Löslichkeit von höchstens 0,05 g HCl / g Lösungsmittel L, vorzugsweise ≤ 0,01 g HCl / g Lösungsmittel L, besonders bevorzugt ≤ 0,008 g HCl / g Lösungsmittel L aufweist.

Besonders bevorzugt enthält das Lösungsmittel L in der Ausführungsform (b)
(i) 20 bis 100 Gew.-%, vorzugsweise 80 bis 100 Gew.-%, besonders bevorzugt 100 Gew.-% (bezogen auf L) einer oder mehrerer Lösungsmittelkomponenten L_{X} gewählt aus der Gruppe bestehend aus linearen oder verzweigten C₅-C₂₀-Alkanen, C₅-C₂₀-Cycloalkanen, Petrolethern und anderen flüssigen Gemischen der genannten gesättigten Kohlenwasserstoffe; und
(ii) 0 bis 80 Gew.-%, vorzugsweise 0 bis 20 Gew.-%, besonders bevorzugt 0 Gew.-% (bezogen auf L) einer oder mehrerer Lösungsmittelkomponenten L_{Y} gewählt aus der Gruppe bestehend aus Benzol, Toluol, o-Xylol, m-Xylol, p-Xylol, Xylol-Isomerengemische, Ethylbenzol und Cumol;
wobei die Summe der Lösungsmittelkomponenten L_{X} und L_{Y} den Wert 100 Gew.-% (bezogen auf L) nicht übersteigt.

Vorzugsweise sind die einen oder mehreren Lösungsmittelkomponenten L_{X} gewählt aus der Gruppe bestehend aus gesättigten Kohlenwasserstoffen, deren Siedepunkte bei Normaldruck im Bereich von 100 bis 200 °C, vorzugsweise 120 bis 180 °C liegen. Dadurch kann die im Laufe der Umsetzung entstehende HCl destillativ aus dem Reaktionsgemisch entfernt oder zumindest abgereichert werden und das Lösungsmittel zu einem späteren Zeitpunkt destillativ entfernt werden.

Bevorzugte Lösungsmittelkomponenten L_{X} sind lineare oder verzweigte C₈-C₁₂-Alkane, deren Gemische und Isomere.

Bevorzugte Lösungsmittelkomponenten L_{Y} sind Toluol, o-Xylol, m-Xylol, p-Xylol, Xylol-Isomerengemische.

Üblicherweise wird das erfindungsgemäße Verfahren folgendermaßen durchgeführt:
Allgemein werden die einen oder mehreren Phenole der Formel (II) mit S₂Cl₂ (III) in dem Lösungsmittel L bei einer Temperatur im Bereich von -20 bis 200 °C über einen Zeitraum von 1 bis 72 h, vorzugsweise 2 bis 48 h, besonders bevorzugt 3 bis 24 h miteinander umgesetzt.

In der Ausführungsform (a) erfolgt die Umsetzung bevorzugt bei einer Temperatur im Bereich von -20 bis 100 °C, besonders bevorzugt -5 bis 80 °C und ganz besonders bevorzugt 5 bis 80 °C. Durch die vergleichsweise (siehe US 3,968,062) geringen Temperaturen wird der Sublimation von nicht reagiertem Phenol der Formel (II) entgegengewirkt.

In der Ausführungsform (b) erfolgt die Umsetzung bevorzugt bei einer Temperatur im Bereich von 0 bis 200 °C, besonders bevorzugt 25 bis 150 °C.

Das erfindungsgemäße Verfahren wird vorzugsweise bei Atmosphärendruck durchgeführt. Unter Atmosphärendruck versteht man im Rahmen der Erfindung einen Druck im Bereich von 1003 bis 1023 mbar, bevorzugt 1013 mbar.

In der Ausführungsform (b) erfolgt die Umsetzung bevorzugt auch bei einem Druck im Bereich von 1 bis 1023 mbar, besonders bevorzugt 200 bis 1023 mbar und ganz besonders bevorzugt 700 bis 1023 mbar.

Wird das Verfahren gemäß der Ausführungsform (b) bei erhöhter Temperatur und/oder vermindertem Druck durchgeführt, so kann die im Laufe der Umsetzung entstehende HCl destillativ aus dem Reaktionsgemisch entfernt oder zumindest abgereichert werden.

Die Durchführung des erfindungsgemäßen Verfahrens erfolgt vorzugsweise unter Inertgasatmosphäre. Bevorzugt als Inertgase sind Argon, Stickstoff, Kohlendioxid. Besonders bevorzugt ist Argon. Besonders bevorzugt ist auch Stickstoff, insbesondere im Hinblick auf eine großtechnische Anwendung. Das erfindungsgemäße Verfahren kann auch an trockener Luft durchgeführt werden.

Die Aufarbeitung der Reaktionsgemische erfolgt beispielsweise durch Filtration, wässrige Aufarbeitung und/oder Destillation.

Im Allgemeinen fallen die Produkte als zähe Öle oder Feststoffe an, die zum Beispiel unter vermindertem Druck (vorzugsweise 10⁻⁵ bis 100 mbar, besonders bevorzugt 10⁻⁴ bis 10 mbar) und/oder bei erhöhter Temperatur (vorzugsweise 120 bis 250 °C, besonders bevorzugt 150 bis 230 °C) von restlichem Lösungsmittel und/oder nicht umgesetztem Phenol befreit werden können.

Fallen die Produkte als Feststoffe an, so kann die Reinigung beispielsweise auch durch Umkristallisieren oder Digerieren erfolgen.

Die Produkte können zum Beispiel auch durch Kristallisation oder Fällung gereinigt werden.

Die erfindungsgemäß hergestellten Diphenoldisulfide (Verbindungen der Formel (I) mit n = 0) können auch durch Vakuumdestillation gereinigt werden.

Bevorzugt liegen die Ausbeuten in einem Bereich von 50 bis 99,8%, besonders bevorzugt 70 bis 98%.

Ein weiterer Gegenstand der Erfindung ist ein Polyphenoldisulfid der Formel (I), erhältlich durch eines der erfindungsgemäßen Verfahren, wobei n ≥ 1 ist.

Erfindungsgemäß sind in einem solchen Polyphenoldisulfid mit n ≠ 0 höchstens 50%, vorzugsweise ≤ 35%, besonders bevorzugt ≤ 20% der in der Formel (I) enthaltenen (n+1) Disulfidbrücken durch eine Mono- oder Oligosulfidbrücke ersetzt.

Weiterhin Gegenstand der Erfindung ist ein Flammschutzsystem, enthaltend
i) mindestens ein erfindungsgemäßes Polyphenoldisulfid der Formel (I); und
ii) mindestens eine halogenfreie organische Phosphorverbindung mit einem Phosphorgehalt im Bereich von 0,5 bis 40 Gew.-%, bezogen auf die Phosphorverbindung.

Ein weiterer Gegenstand der Erfindung ist eine Polymerzusammensetzung, enthaltend ein oder mehrere Polymere und das erfindungsgemäße Flammschutzsystem.

Weiterhin Gegenstand der Erfindung ist ein Diphenoldisulfid der Formel (la), 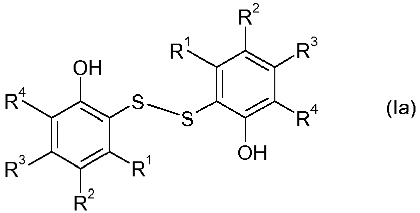 wobei die Symbole R¹, R², R³ und R⁴ folgende Bedeutungen haben:
- R²: ist gleich oder verschieden C₆-C₁₂-Aryl
- R¹, R³, R⁴: sind gleich oder verschieden C₁-C₁₈-Alkyl, C₂-C₁₈-Alkenyl, C₂-C₁₈-Alkinyl, C₆-C₁₂-Aryl, C₃-C₁₀-Cycloalkyl, C₆-C₁₂-Aryl-C₁-C₁₈-Alkyl, eine Heteroarylgruppe, die ein oder mehrere Heteroatome aus der Gruppe N, O und S enthält, O-(C₁-C₁₈)-Alkyl, O-(C₁-C₁₈)-Alkenyl, O-(C₂-C₁₈)-Alkinyl, O-(C₆-C₁₂)-Aryl, O-(C₃-C₁₀)-Cycloalkyl, (C₆-C₁₂)-Aryl-(C₁-C₁₈)-Alkyl-O, S-(C₁-C₁₈)-Alkyl, S-(C₂-C₁₈)-Alkenyl, S-(C₂-C₁₈)-Alkinyl, S-(C₆-C₁₂)-Aryl, S-(C₃-C₁₀)-Cycloalkyl, (C₆-C₁₂)-Aryl-(C₁-C₁₈)-Alkyl-S, OH, F, Cl, Br oder H.

Die bevorzugten Bedeutungen der Symbole R¹, R³ und R⁴ in der Formel (Ia) entsprechen den jeweils bevorzugten Bedeutungen dieser Symbole in der Formel (I).

Bevorzugt sind Diphenoldisulfide der Formel (Ia), in denen alle Symbole die bevorzugten Bedeutungen haben.

Die besonders bevorzugten Bedeutungen der Symbole R¹, R³ und R⁴ in der Formel (Ia) entsprechen den jeweils besonders bevorzugten Bedeutungen dieser Symbole in der Formel (I).

Besonders bevorzugt sind Diphenoldisulfide der Formel (Ia), in denen alle Symbole die besonders bevorzugten Bedeutungen haben.

Die ganz besonders bevorzugten Bedeutungen der Symbole R¹, R³ und R⁴ in der Formel (Ia) entsprechen den jeweils ganz besonders bevorzugten Bedeutungen dieser Symbole in der Formel (I).

Ganz besonders bevorzugt sind Diphenoldisulfide der Formel (Ia), in denen alle Symbole die ganz besonders bevorzugten Bedeutungen haben.

Weiterhin ganz besonders bevorzugt ist auch die Verbindung der Formel (Ia) Di-(para-phenylphenol)disulfid:

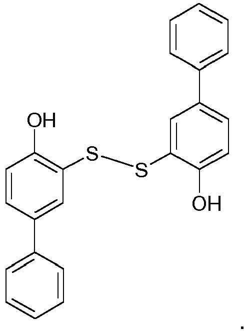

Weiterhin Gegenstand der Erfindung ist Di-(ortho-phenylphenol)disulfid:

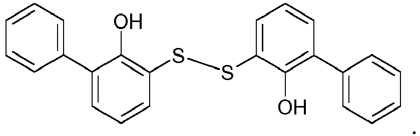

Ein weiterer Gegenstand der Erfindung ist ein Polyphenoldisulfid der Formel (Ib), 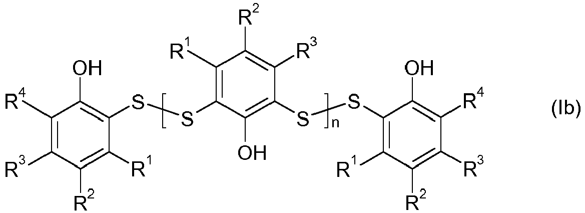 wobei die Symbole R¹, R², R³, R⁴ und n folgende Bedeutungen haben:
- R²: ist gleich oder verschieden C₆-C₁₂-Aryl;
- R¹, R³, R⁴: sind gleich oder verschieden C₆-C₁₂-Aryl oder H;
- n: ist eine ganze Zahl von 0 bis 1000, vorzugsweise eine ganze Zahl von 1 bis 1000.

Bevorzugt haben die Symbole in der Formel (Ib) folgende Bedeutungen:
- R⁴: ist bevorzugt gleich oder verschieden C₆-C₁₂-Aryl oder H.
- R¹, R³: sind bevorzugt H.
- n: ist bevorzugt eine ganze Zahl von 0 bis 500, vorzugsweise eine ganze Zahl von 1 bis 500.

Bevorzugt sind Polyphenoldisulfide der Formel (Ib), in denen alle Symbole die bevorzugten Bedeutungen haben.

Besonders bevorzugt haben die Symbole in der Formel (Ib) folgende Bedeutungen:
- R²: ist besonders bevorzugt gleich oder verschieden C₆-C₁₂-Aryl.
- R¹, R³, R⁴: sind besonders bevorzugt H.
- n: ist besonders bevorzugt eine ganze Zahl von 0 bis 250, vorzugsweise eine ganze Zahl von 1 bis 250.

Besonders bevorzugt sind Polyphenoldisulfide der Formel (Ib), in denen alle Symbole die besonders bevorzugten Bedeutungen haben.

Ganz besonders bevorzugt haben die Symbole in der Formel (Ib) folgende Bedeutungen:
- R²: ist ganz besonders bevorzugt Phenyl.
- R¹, R³, R⁴: sind ganz besonders bevorzugt H.
- n: ist ganz besonders bevorzugt eine ganze Zahl von 0 bis 150, vorzugsweise eine ganze Zahl von 1 bis 150.

Ganz besonders bevorzugt sind Polyphenoldisulfide der Formel (Ib), in denen alle Symbole die ganz besonders bevorzugten Bedeutungen haben.

Weiterhin ganz besonders bevorzugt ist die Verbindung Poly-(para-phenylphenoldisulfid):

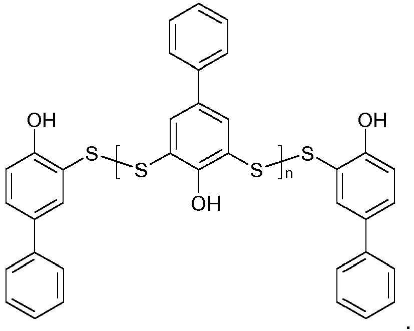

Die nach dem erfindungsgemäßen Verfahren hergestellten Polyphenoldisulfide eignen sich beispielsweise als Flammschutzsynergisten in Polymeren. Bevorzugt ist die Verwendung in Schaumstoffen, insbesondere in auf Polystyrol basierenden Schaumstoffen.

Der erfindungsgemäß hergestellte Flammschutzsynergist wird in Mischung mit einem oder mehreren Flammschutzmitteln und gegebenenfalls mit weiteren Synergisten für die Herstellung flammhemmend ausgerüsteter (bzw. flammgeschützter) Polymere, insbesondere thermoplastischer Polymere, verwendet. Hierfür werden die Flammschutzmittel und Synergisten vorzugsweise physikalisch mit dem entsprechenden Polymer in der Schmelze vermischt und dann entweder als Polymermischung zunächst fertig konfektioniert und dann in einem zweiten Verfahrensschritt zusammen mit dem gleichen oder einem anderen Polymer weiterverarbeitet. Alternativ ist im Falle von Styrolpolymeren auch der Zusatz der Flammschutzmittel und Synergisten vor, während und/oder nach der Herstellung durch Suspensionspolymerisation bevorzugt.

Als Polymer können beispielsweise geschäumte oder ungeschäumte Styrolpolymere, einschließlich ABS, ASA, SAN, AMSAN, SB und HIPS Polymere, Polyimide, Polysulfone, Polyolefine wie Polyethylen und Polypropylen, Polyacrylate, Polyetheretherketone, Polyurethane, Polycarbonate, Polyphenylenoxide, ungesättigte Polyesterharze, Phenolharze, Polyamide, Polyethersulfone, Polyetherketone und Polyethersulfide, jeweils einzeln oder in Mischung als Polymerblends eingesetzt werden.

Bevorzugt sind thermoplastische Polymere wie geschäumte oder ungeschäumte Styrolhomopolymere und -copolymere jeweils einzeln oder in Mischung als Polymerblends.

Erfindungsgemäß umfasst der Begriff Styrolpolymer Polymere auf Basis von Styrol, alpha-Methylstyrol oder Mischungen von Styrol- und alpha-Methylstyrol; analog gilt dies für den Styrolanteil in SAN, AMSAN, ABS, ASA, MBS und MABS (siehe unten). Erfindungsgemäße Styrolpolymere basieren auf mindestens 50 Gew.-Teilen Styrol und/oder alpha-Methylstyrol Monomeren.

Bevorzugt werden als Styrolpolymere glasklares Polystyrol (GPPS), Schlagzähpolystyrol (HIPS), anionisch polymerisiertes Polystyrol oder Schlagzähpolystyrol (A-IPS), Styrol-alpha-Methylstyrol-copolymere, Acrylnitril-Butadien-Styrolpolymerisate (ABS), Styrol-Acrylnitril-Copolymere (SAN), Acrylnitril-alpha-Methylstyrol-Copolymere (AMSAN), Acrylnitril-Styrol-Acrylester (ASA), Methylacrylat-Butadien-Styrol (MBS), Methylmethacrylat-Acrylnitril-Butadien-Styrol (MABS)-polymerisate oder Mischungen davon oder mit Polyphenylenether (PPE) eingesetzt.

Die genannten Styrolpolymere können zur Verbesserung der mechanischen Eigenschaften oder der Temperaturbeständigkeit gegebenenfalls unter Verwendung von Verträglichkeitsvermittlern mit thermoplastischen Polymeren, wie Polyamiden (PA), Polyolefinen wie Polypropylen (PP) oder Polyethylen (PE), Polyacrylaten wie Polymethylmethacrylat (PMMA), Polycarbonat (PC), Polyestern wie Polyethylenterephthalat (PET) oder Polybutylenterephthalat (PBT), Polyethersulfonen (PES), Polyetherketonen oder Polyethersulfiden (PES) oder Mischungen davon in Anteilen von insgesamt bis maximal 30 Gew.-Teilen, bevorzugt im Bereich von 1 bis 10 Gew.-Teilen, bezogen auf 100 Gew.-Teile der Polymerschmelze, enthalten. Des Weiteren sind Mischungen in den genannten Mengenbereichen auch mit zum Beispiel hydrophob modifizierten oder funktionalisierten Polymeren oder Oligomeren, Kautschuken wie Polyacrylaten oder Polydienen, zum Beispiel Styrol-Butadien-Blockcopolymeren oder biologisch abbaubaren aliphatischen oder aliphatisch/aromatischen Copolyestern möglich.

Bevorzugt sind flammgeschützte Polymerschaumstoffe, insbesondere auf Basis von Styrolpolymeren, vorzugsweise EPS und XPS.

Die expandierbaren Styrolpolymere (EPS) können entweder im Extrusionsverfahren oder mittels Suspensionspolymerisation in wässriger Suspension in Gegenwart eines Flammschutzmittels, des erfindungsgemäßen Synergisten und eines organischen Treibmittels hergestellt werden.

Die flammgeschützten Polymerschaumstoffe weisen bevorzugt eine Dichte im Bereich von 5 bis 200 kg/m³, besonders bevorzugt im Bereich von 10 bis 50 kg/m³, auf und sind bevorzugt zu mehr als 80%, besonders bevorzugt zu 90 bis 100% geschlossenzellig.

Die Erfindung wird durch die nachfolgenden Beispiele näher erläutert.

### Beispiele

### A. Synthesebeispiele

### Beispiel 1

### Synthese von Di-(tert.-butylphenol)disulfid in DMF:

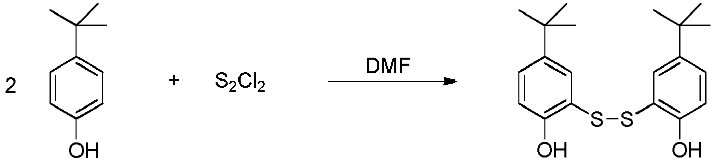

In einen sorgfältig getrockneten Dreihalskolben, der mit Innenthermometer, Rührer und Inertgasüberleitung sowie einem Kühlbad ausgerüstet ist, werden 22,53 g (0,15 mol) p-*tert*.-Butylphenol gegeben. Dann wird die Apparatur mit Argon gefüllt. Anschließend werden 80 ml abs. DMF zugegeben. Die erhaltene Lösung des t-Butylphenols wird auf ca. 0°C gekühlt. Dann werden 10 g (0,074 mol) Dischwefeldichlorid während einer Stunde tropfenweise zugegeben, wobei die Temperatur bei 0-3°C gehalten wird. Nach Beendigung der Reagenzzugabe lässt man die Temperatur im Verlauf von 15 h auf ca. 25°C ansteigen. Dann wird die gelbe Lösung 3 h auf 45-50°C erwärmt. Anschließend wird eine Vakuumdestillation durchgeführt. Bei 185°C / 0,01 mbar destilliert das Di-(*tert*.-butylphenol)disulfid als orange-gelbes Öl, das zu einem glasartigen Feststoff erstarrt. Dieser wird in 70 ml siedendem n-Pentan gelöst. Nach Abkühlen auf ca. -20°C scheidet sich reines Di-(*tert*.-butylphenol)disulfid als hellgelber, kristalliner Feststoff aus (Ausbeute: 12,5 g; 64%).

### Beispiel 2

### Synthese von Di-(tert.-butylphenol)disulfid in 1,3-Dimethyl-2-imidazolidinon:

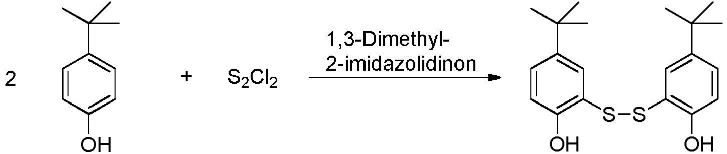

In einen sorgfältig getrockneten Dreihalskolben, der mit Innenthermometer, Rührer und Inertgasüberleitung sowie einem Kühlbad ausgerüstet ist, werden 22,53 g (0,15 mol) p-*tert*.-Butylphenol gegeben. Dann wird die Apparatur mit Argon gefüllt. Anschließend werden 80 ml wasserfreies 1,3-Dimethyl-2-imidazolidinon zugegeben. Die erhaltene Lösung des t-Butylphenols wird auf ca. 0°C gekühlt. Dann werden 10 g (0,074 mol) Dischwefeldichlorid während einer Stunde tropfenweise zugegeben, wobei die Temperatur bei 0-3°C gehalten wird. Nach Beendigung der Reagenzzugabe lässt man die Temperatur im Verlauf von 15 h auf ca. 25°C ansteigen. Dann wird die gelbe Lösung 3 h auf 45°C erwärmt. Anschließend wird eine fraktionierte Vakuumdestillation durchgeführt. Bei 185°C / 0,01 mbar destilliert Di-(*tert*.-butylphenol)disulfid als orange-gelbes Öl, das zu einem glasartigen Feststoff erstarrt. Auf diese Weise werden 16 g Di-(*tert*-butylphenol)disulfid mit einer Reinheit von ca. 95% erhalten (Ausbeute: 82%).

### Beispiel 3

### Synthese von Di-(tert.-butylphenol)disulfid in 1,3-Dimethyl-3,4,5,6-tetrahydro-2(1H)-pyrimidinon:

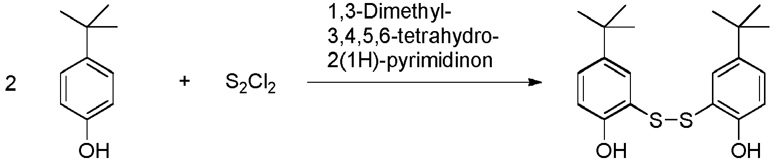

In einen sorgfältig getrockneten Dreihalskolben, der mit Innenthermometer, Rührer und Inertgasüberleitung sowie einem Kühlbad ausgerüstet ist, werden 22,53 g (0,15 mol) p-*tert*.-Butylphenol gegeben. Dann wird die Apparatur mit Argon gefüllt. Anschließend werden 80 ml wasserfreies 1,3-Dimethyl-3,4,5,6-tetrahydro-2(1H)-pyrimidinon zugegeben. Die erhaltene Lösung des *t*-Butylphenols wird auf ca. 0°C gekühlt. Dann werden 10 g (0,074 mol) Dischwefeldichlorid während einer Stunde tropfenweise zugegeben, wobei die Temperatur bei ca. 3°C gehalten wird. Nach Beendigung der Reagenzzugabe lässt man die Temperatur im Verlauf von 15 h auf ca. 25°C ansteigen. Dann wird die gelbe Lösung 5 h auf 45°C erwärmt. Anschließend wird eine fraktionierte Vakuumdestillation durchgeführt. Bei 185°C / 0,01 mbar destilliert Di-(*tert*.-butylphenol)disulfid als orange-gelbes Öl, das zu einem glasartigen Feststoff erstarrt. Auf diese Weise werden 15,5 g Di-(*tert*.-butylphenol)disulfid mit einer Reinheit von ca. 95% erhalten (Ausbeute: 80%).

### Beispiel 4

### Synthese von Di-(tert.-butylphenol)disulfid in DMSO:

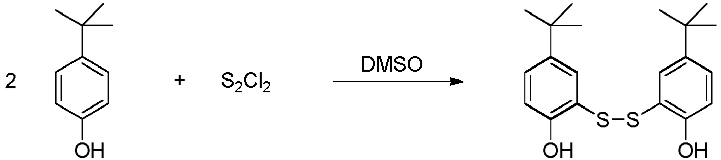

In einen sorgfältig getrockneten Dreihalskolben, der mit Innenthermometer, Rührer und Inertgasüberleitung sowie einem Kühlbad ausgerüstet ist, werden 22,54 g (0,15 mol) p-*tert*.-Butylphenol gegeben. Dann wird die Apparatur mit Argon gefüllt. Anschließend werden 80 ml wasserfreies DMSO zugegeben. Die erhaltene Lösung des *t*-Butylphenols wird auf ca. -3°C gekühlt. Dann wird eine Mischung aus 10,2 g (0,075 mol) Dischwefeldichlorid und 20 ml wasserfeiem DMSO innerhalb einer Stunde unter Rühren zugegeben, wobei die Temperatur bei max. 5°C gehalten wird. Im Verlauf von ca. 20 h lässt man die Temperatur auf ca. 25°C ansteigen, und danach wird die Lösung 4 h auf 50°C erwärmt (Bei der Umsetzung erfolgt keine Freisetzung von HCl-Gas). Nun wird das Lösungsmittel bei ca. 1 mbar abdestilliert, und anschließend wird der Rückstand bei 0,01 mbar auf 180°C erwärmt. Erhalten wird eine zähe Substanz (Menge: 26,4 g), welche ca. 80% Di-(*tert*.-butylphenol)disulfid enthält.

### Beispiel 5

### Synthese von Di-(tert.-pentylphenol)disulfid in DMF:

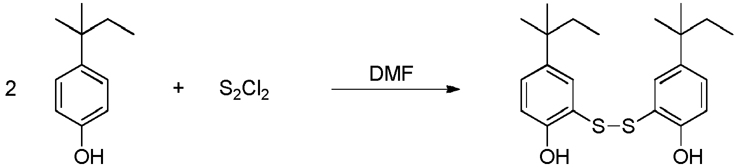

In einen sorgfältig getrockneten Dreihalskolben, der mit Innenthermometer, Rührer und Inertgasüberleitung sowie einem Kühlbad ausgerüstet ist, werden 24,63 g (0,15 mol) p-*tert*.-Pentylphenol gegeben. Dann wird die Apparatur mit Argon gefüllt. Anschließend werden 75 ml abs. DMF zugegeben. Die erhaltene Lösung des p-*tert*-Pentylphenols wird auf ca. -3°C gekühlt. Dann werden 9,5 g (0,07 mol) Dischwefeldichlorid tropfenweise zugegeben, wobei die Temperatur bei 0-3°C gehalten wird. Nach Beendigung der Reagenzzugabe lässt man die Temperatur im Verlauf von 15 h auf ca. 25°C ansteigen. Dann wird die gelbe Lösung 3 h auf 45-50°C erwärmt. Im Anschluss daran werden das DMF und die anderen flüchtigen Bestandteile abdestilliert, wobei die Temperatur bis 170°C erhöht und der Druck auf ca. 0,01 mbar abgesenkt wird. Als Destillationsrückstand verbleibt eine zähe Substanz (Menge: 28,3 g), welche ca. 85% Di-(*tert*.-pentylphenol)disulfid enthält. Reines Di-(*tert*.-pentylphenol)disulfid wird durch Destillation im Feinvakuum isoliert (Siedetemperatur ca. 190°C bei 0,001 mbar). Ausbeute: 17,5 g, 64% (bezogen auf eingesetztes S₂Cl₂).

### Beispiel 6

### Synthese von Poly-(tert.-butylphenoldisulfid) in DMF:

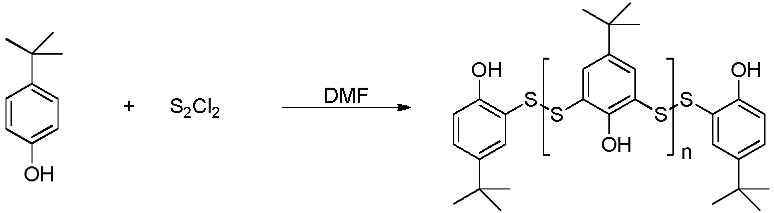

In einen sorgfältig getrockneten Dreihalskolben, der mit Innenthermometer, Rührer und Inertgasüberleitung sowie einem Kühlbad ausgerüstet ist, werden 22,53 g (0,15 mol) p-*tert*.-Butylphenol gegeben. Dann wird die Apparatur mit Argon gefüllt. Anschließend werden 60 ml abs. DMF zugegeben, und es werden 18,9 g (0,14 mol) Dischwefeldichlorid in den Tropftrichter gefüllt. Die erhaltene Lösung des *t*-Butylphenols wird auf ca. 0°C gekühlt. Dann wird im Verlauf von ca. 2 h das Dischwefeldichlorid tropfenweise unter Rühren zugegeben, wobei die Temperatur bei max. 5°C gehalten wird. Im Verlauf von weiteren 10 h lässt man die Temperatur auf ca. 25°C ansteigen. Schließlich wird noch 2 h bei 45-50°C gerührt (Während der Umsetzung ist keine Freisetzung von HCl-Gas zu beobachten). Von der erhaltenen tiefgelben Lösung wird nun das DMF im Vakuum (< 1 mbar) abdestilliert, wobei allmählich bis auf 170°C erwärmt wird. Beim Erkalten wird ein spröder Feststoff erhalten, welcher zerkleinert und in 70 ml Diethylether gelöst wird. Diese Lösung wird in 350ml n-Pentan getropft, wobei sich das Produkt gelartig abscheidet. Die überstehende Lösung wird abdekantiert. Das Produkt wird im Vakuum bei max. ca. 150°C getrocknet. Beim Zerkleinern wird ein hellgelbes Pulver (Menge: 27,9 g) erhalten, das ca. 80% Poly-(*tert*.-butylphenoldisulfid) enthält.

### Beispiel 7

### Synthese von Poly-(tert.-butylphenoldisulfid) in N,N,N',N'-Tetramethylharnstoff:

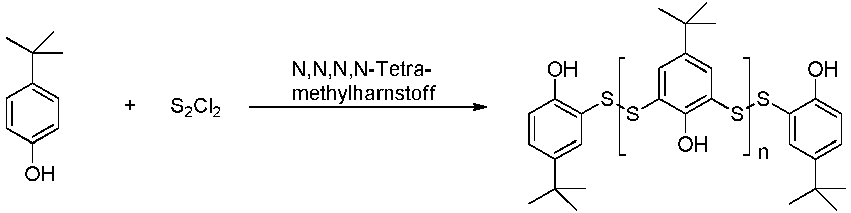

In einen sorgfältig getrockneten Dreihalskolben, der mit Innenthermometer, Rührer und Inertgasüberleitung sowie einem Kühlbad ausgerüstet ist, werden 22,53 g (0,15 mol) p-*tert*.-Butylphenol gegeben. Dann wird die Apparatur mit Argon gefüllt. Anschließend werden 60 ml wasserfreier N,N,N',N'-Tetramethylharnstoff zugegeben. Die erhaltene Lösung des t-Butylphenols wird auf ca. 0°C gekühlt. Dann werden im Verlauf von ca. 2 h 18,9 g (0,14 mol) Dischwefeldichlorid tropfenweise unter Rühren zugegeben, wobei die Temperatur bei max. 3°C gehalten wird. Im Verlauf von weiteren 10 h lässt man die Temperatur auf ca. 25°C ansteigen. Schließlich wird noch 2 h bei 45-50°C gerührt (Während der Umsetzung ist keine Freisetzung von HCl-Gas zu beobachten). Von der erhaltenen tiefgelben Lösung wird nun der N,N,N',N'-Tetramethylharnstoff im Vakuum (0,1 mbar) abdestilliert, wobei allmählich bis auf 180°C erwärmt wird. Beim Erkalten wird ein spröder Feststoff erhalten, welcher zerkleinert und in 70 ml Diethylether gelöst wird. Diese Lösung wird in 350ml n-Pentan getropft, wobei sich das Produkt gelartig abscheidet. Die überstehende Lösung wird abdekantiert. Das Produkt wird im Vakuum bei max. ca. 190°C getrocknet. Beim Zerkleinern wird ein hellgelbes Pulver (Menge: 28,1 g) erhalten, das ca. 85% Poly-(*tert*.-butylphenoldisulfid) enthält.

### Beispiel 8

### Synthese von Poly-(tert.-pentylphenoldisulfid) in DMF / Toluol:

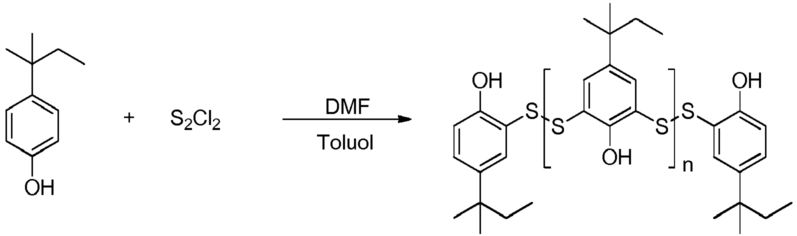

In eine sorgfältig getrocknete Apparatur werden 0,5 mol (82,13 g) *tert*-Pentylphenol, 1,00 mol (73,1 g) trockenes DMF sowie 250 ml abs. Toluol gegeben. Nach dem Auflösen des *tert*-Pentylphenols wird die Lösung auf ca. 10°C gekühlt. Dann werden unter Rühren 0,46 mol (62,11 g) Dischwefeldichlorid zugetropft, wobei die Temperatur bei 10-12°C gehalten wird. Nach dem Ende der Reagenzzugabe lässt man die Temperatur im Verlauf von ca. 5h auf Raumtemperatur ansteigen. Es entsteht eine Suspension, die aber gut rührbar bleibt. Das Rühren bei ca. 22°C wird noch 15 h fortgesetzt. Dann wird die Temperatur auf 50°C erhöht und nach 3 h auf 75°C gesteigert. Bei dieser Temperatur wird noch 2 h gerührt. Danach wird der warme Kolbeninhalt in einen Scheidetrichter überführt. Der Hauptteil des DMF-Hydrochlorids scheidet sich als untere Phase ab und wird warm abgetrennt. Die Toluolphase wird dreimal mit je 100ml Wasser gewaschen. Beim dritten Waschen wird 0,5 ml 25%-ige Ammoniaklösung zugegeben. Danach wird die Toluol-Lösung 1 h mit 10 ml 15%-iger Wasserstoffperoxid-Lösung bei 50°C gerührt. Nach dem Abtrennen des Wasserstoffperoxids wird mit 100 ml Wasser, dem ein Tropfen Salzsäure zugesetzt wird, extrahiert. Anschließend wird die gelbe Lösung über Natriumsulfat getrocknet. Nach dem Abfiltrieren des Trockenmittels werden das Toluol und die anderen die flüchtigen Bestandteile in eine Kühlfalle abdestilliert. Hierbei wird die Temperatur allmählich erhöht und gleichzeitig wird der Druck langsam gesenkt. Bei einer Temperatur von 170°C wird ein Druck von ca. 0,01 mbar erreicht. Nun wird 30 min bei 175-180°C / 0,01 mbar gerührt und weitere 30 min bei 190-195°C / 0,01 mbar. Das Rühren im Vakuum wird noch 30 min fortgesetzt, wobei die Temperatur bis auf 210°C gesteigert wird. Während der Vakuumdestillation fällt ca. 1 g hochsiedendes Destillat an, das verworfen wird. Nach der Destillation wird die Schmelze ausgegossen und nach ihrem Erstarren zerkleinert, wobei ein gelbes, nahezu geruchloses Pulver (Menge: 102,3 g) erhalten wird, das zu ca. 70% Poly-(*tert*.-pentylphenoldisulfid) besteht.

### Beispiel 9

### Synthese von Poly-(tert.-pentylphenoldisulfid) in N,N,N',N'-Tetramethylharnstoff:

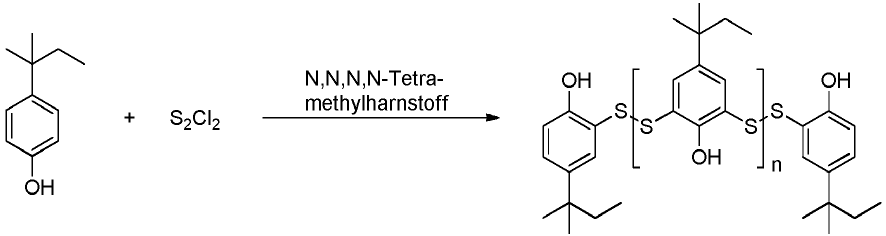

In einen sorgfältig getrockneten Dreihalskolben, der mit Innenthermometer, Rührer und Inertgasüberleitung sowie einem Kühlbad ausgerüstet ist, werden 73,91 g (0,45 mol) p-*tert*.-Pentylphenol gegeben. Dann wird die Apparatur mit Argon gefüllt. Anschließend werden 200 ml wasserfreier N,N,N',N'-Tetramethylharnstoff zugegeben, und es werden 56,7 g (0,42 mol) Dischwefeldichlorid in den Tropftrichter gefüllt. Die erhaltene Lösung des p-*tert*-Pentylphenols wird auf ca. 0°C gekühlt. Dann wird im Verlauf von ca. 2 h das Dischwefeldichlorid tropfenweise unter Rühren zugegeben, wobei die Temperatur bei max. 5°C gehalten wird. Im Verlauf von weiteren 20 h lässt man die Temperatur auf ca. 25°C ansteigen. Anschließend wird 3 h bei 45-50°C gerührt und weitere 2 h bei 70°C (Während der Umsetzung ist keine Freisetzung von HCl-Gas zu beobachten). Die gelbe Lösung wird auf ca. 50°C gekühlt. Dann werden 60 ml Wasser im Verlauf von 20 min unter kräftigem Rühren zugegeben, wobei sich eine zähe Substanz ausscheidet. Nach dem Abdekantieren der überstehenden Lösung wird diese Substanz viermal mit Wasser gewaschen. Danach wird sie auf 190°C erhitzt, wobei der Druck allmählich bis auf 0,01 mbar gesenkt wird. Es wird 30 min bei 190°C / 0,01 mbar gerührt, wobei geringe Mengen verdampfbarer Bestandteile abdestillieren. Schließlich wird die so erhaltene Schmelze ausgegossen und nach ihrem Erstarren zu einem Pulver (Menge: 95,5 g) zerkleinert, welches etwa 85% Poly-(*tert*.-pentylphenoldisulfid) enthält.

### Beispiel 10

### Synthese von Poly-(tert.-pentylphenoldisulfid) in N,N,N',N'-Tetramethylharnstoff / Toluol:

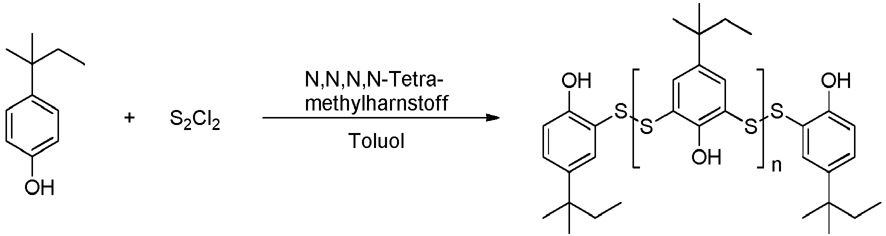

In einen sorgfältig getrockneten Dreihalskolben, der mit Innenthermometer, Rührer und Inertgasüberleitung sowie einem Kühlbad ausgerüstet ist, werden 42,88 g (0,26 mol) p-*tert*.-Pentylphenol gegeben. Dann wird die Apparatur mit Argon gefüllt. Anschließend werden 56 g wasserfreier N,N,N',N'-Tetramethylharnstoff sowie 175 ml abs. Toluol zugegeben. Nach dem Auflösen des p-*tert*-Pentylphenols wird auf ca. 0°C gekühlt. Dann werden 32,9 g (0,244 mol) Dischwefeldichlorid innerhalb 1 h tropfenweise unter Rühren zugegeben, wobei die Temperatur bei max. 3°C gehalten wird. Im Verlauf von weiteren 20 h lässt man die Temperatur auf ca. 25°C ansteigen. Anschließend wird 2,5 h bei 45-50°C gerührt, 1,5 h bei 60°C und weitere 2 h bei 70°C (Während der Umsetzung ist keine Freisetzung von HCl-Gas zu beobachten). Danach wird der noch warme Kolbeninhalt, der aus zwei flüssigen Phasen besteht, in einen Scheidetrichter überführt. Die obere Phase (ca. 100 ml) enthält nur geringe Mengen niedermolekularer Substanzen und wird nach dem Abdestillieren des Toluols verworfen. Die untere Phase (ca. 200 ml) enthält das Produkt sowie Toluol und Hydrochloride des N,N,N',N'-Tetramethylharnstoffs. Diese Bestandteile werden abdestilliert, wobei die Temperatur langsam bis auf 190°C erhöht und der Druck allmählich auf 0,01 mbar abgesenkt wird. Das Rühren bei 190°C / 0,01 mbar wird noch 30 min fortgesetzt, wobei geringe Mengen verdampfbarer Substanzen abdestillieren. Anschließend wird die Schmelze ausgegossen. Der beim Erkalten erhaltene Feststoff wird in 100 ml Ethanol gelöst, dem 1,5 ml Wasserstoffperoxid-Lösung zugesetzt werden. Diese Lösung wird 2 h bei 50-55°C gerührt. Anschließend werden 75 ml Wasser unter Rühren zugefügt, wobei sich eine gelartige Substanz ausscheidet. Nach dem Abdekantieren der überstehenden Flüssigkeit werden die flüchtigen Bestandteile abdestilliert, wobei die Temperatur im Vakuum auf 195°C erhöht und der Druck auf 0,01 mbar abgesenkt wird. Es wird noch 30 min unter diesen Bedingungen gerührt. Nach dem Ausgießen der Schmelze, Erstarren und Zerkleinern wird ein gelbes Pulver (Menge: 55,3 g) erhalten. Dieses enthält ca. 80% Poly-(*tert*.-pentylphenoldisulfid).

### Vergleich von erfindungsgemäß hergestellten Polyphenoldisulfiden mit Polyphenoldisulfiden des Standes der Technik:

**Tabelle 1: Zusammensetzung und Temperaturstabilität von erfindungsgemäß hergestelltem Polyphenoldisulfiden (Beispiele) und von Vergleichsbeispielen**

| **Beispiel** | Produktmuster | Anteil an dimeren und polymeren Phenoldisulfideinheiten^{a} | Anteil an Monosulfideinheiten^{a} | Gehalt an elementarem Schwefel^{b} | Temperaturstabilität (Temperatur bei 2% Massenverlust)^{c} | Chlor gehalt (kovalent)^{d} |
|---|---|---|---|---|---|---|
| **V1** | Kommerzielles VULTAC TB7 (Poly-tert.-butylphenoldisulfid) | < 50% | 45% | >3% | 275 °C | 1,1% |
| **V2** | Kommerzielles VULTAC 3 (Poly-tert.-pentylphenoldisulfid) | < 50% | 45% | >3% | 303 °C | 1,1% |
| **V3** | Poly-tert.-butylphenoldisulfid, synthetisiert in Trichlorethan^{e} | < 50% | 45% | >3% | 273 °C | 1,8% |
| **B1** | Poly-tert.-butylphenoldisulfid, synthetisiert in DMF gemäß Beispiel 6 | >80% | < 20% | < 1% | 299 °C | 0,8% |
| **B2** | Poly-tert.-pentylphenoldisulfid, synthetisiert in DMF/Toluol gemäß Beispiel 8 | >80 | <20 | <1% | 315 °C | 0,3% |

| | | | | | | |
|---|---|---|---|---|---|---|
| ^{a}bestimmt über 1H-NMR-spektroskopische Analyse unter Verwendung von DMSO-d₆ als Lösungsmittel; Anteil angegeben in Gew.-%, bezogen auf das Produktmuster; ^{b}Gehalt angegeben in Gew.-%, bezogen auf das Produktmuster; ^{c}bestimmt über thermogravimetrische Analyse (Gerät: Mettler Toledo TGA/SDTA 851, unter N₂, 20-800 °C, Heizrate 10 K/min); ^{d}Gehalt angegeben in Gew.-%, bezogen auf das Produktmuster; ^{e}synthetisiert nach der in US 3,968,062 beschriebenen Vorschrift unter Verwendung von Trichlorethan als Lösungsmittel. | | | | | | |

### Analysenmethode Schwefelbestimmung:

### Prinzip

Verbrennung der Probe bei ca. 1050°C im Helium-Trägergasstrom mit zudosiertem Sauerstoff. Als Verbrennungszuschlag wird der Probe ein Gemisch aus V₂O₅ + WO₃ (2:1 m/m) zugesetzt. Unter diesen Bedingungen reagiert der Schwefel zu SO₂/SO₃. Anschließend erfolgt eine Reduktion des Schwefeltrioxids an heißem, metallischem Kupfer zu SO₂. Danach wird das Verbrennungsgas mittels Sicapent getrocknet. Die Auftrennung der Komponenten erfolgt an einer Chromatographietrennsäule. Detektion: Direkt als SO₂ über Wärmeleitfähigkeit (WLD). Auswertung: Die Auswertung erfolgt mittels PC und spezieller Auswertesoftware.

### Reagenzien

- Kupfer, Drahtform, z.B. Fa. IVA
- Wolframoxid auf Aluminiumoxid (15:85), z.B. Fa. BASF
- Vanadiumpentoxid, z.B. Fa. Merck
- Wolframoxid, z.B. Fa. Merck
- Sicapent (Trocknungsmittel), z.B. Fa. Merck
- Kapseln / Falttiegel aus Reinzinn, z.B. Fa. IVA
- Quarzsplitter / Quarzwolle, z.B. Fa. BASF
- Helium 4.6, z.B. Fa. Air Liquide
- Sauerstoff, z.B. Fa. Air Liquide

### Geräte

- EuroVector EA, Fa. Eurovector
- Waage, geeignet für Mikrobereich, z.B. Fa. Mettler MT5

### Durchführung

Einwaage: 1 - 10 mg der Probe werden auf 0,001 mg genau in eine Zinnkapsel eingewogen. Zusätzlich werden ca. 15 mg Zuschlagsgemisch V₂O₅ + WO₃ (2:1 m/m) zugegeben und die Kapsel anschließend verschlossen. Verbrennung: Über eine Probenaufgabevorrichtung erfolgt die Einschleusung in ein mit metallischem Kupfer und Quarzsplittern gefülltes und auf ca. 1050°C erhitztes Quarzrohr. Der Schwefel wird dabei zu SO₂/SO₃ umgesetzt. Anschließend erfolgt eine Reduktion des Schwefeltrioxids an heißem, metallischem Kupfer zu SO₂. Gastrocknung: Das Verbrennungsgas wird über Sicapent geleitet, um das Gas zu trocknen. Messprinzip Eurovector EA: Bestimmung von SO₂ durch Wärmeleitfähigkeitsdetektion (WLD). 7.5 Messparameter: He-Trägergasdruck: ca. 100 kPa He-Spülung: ca. 80 ml/min Pyrolysetemperatur: ca. 1050°C Säulentemperatur: ca. 60°C Detektortemperatur: ca. 60°C

### Kalibration / Berechnung

Es werden verschiedene Referenzmaterialien unterschiedlichen Schwefelgehalts analysiert. Aus den gemessenen Peakflächen und den absoluten Gehalten an Schwefel wird eine lineare Regression durchgeführt.

### Analysenmethode Chlorgehalt:

### Prinzip

Verbrennung der Probe im Sauerstoffstrom und anschließender potentiometrischer Titration des Bromids und Chlorids mit Silbernitrat-Maßlösung. Die Methode ist geeignet für Proben mit einem Chlorgehalt von w(Cl) > 0,5 g/100g

### Reagenzien

- Hydrazinsulfat N₂H₆SO₄, z.B. Fa. Merck
- Wässrige Hydrazinsulfat-Lösung (w(N₂H₆SO₄) = ∼2 g/100g)
- Halogenfreier Sauerstoff, z.B. Fa. BASF
- Zinnkapseln, z.B. Fa. Lüdi AG, Flawil (CH)
- Wässrige Silbernitrat-Maßlösung (c(AgNO₃) = 0,001 mol/l, z.B. Fa. Merck)
- Essigsäure = -75 %ig, z.B. Fa. Merck
- Natriumbromidlösung c(NaBr) = 0,001 mol/l, z.B. Fa. Merck
- Natriumchloridlösung c(NaCl) = 0,001 mol/l, z.B. Fa. Merck
- deionisiertes Wasser, z.B. Fa. BASF

### Geräte

- AQF 100 mit Autosampler, Fa. a1-envirotech
- Mikrowaage z.B. ME5, Fa. Sartorius
- Titrierstand, z.B. 794 Basic Titrino, Fa. Metrohm
- Autosampler zu Titrierstand, z.B. 815 Autosampler, Fa. Metrohm
- Dosino, z.B. 800 Dosino, Fa. Metrohm
- Spülpumpe, z.B. 772 Pump Unit, Fa. Metrohm
- Glasabsorptionsgefäß mit Fritte, z.B. Eigenanfertigung, Fa. BASF
- Silberelektrode + Sulfit-Überzug, z.B. Titrode 6.0430.100, Fa. Metrohm
- Übliche Laborgeräte

### Durchführung

### Verbrennung und Messung

Die Probe (max. 15 mg bei rein organischer Matrix) wird auf 0,001 mg genau in eine Zinnkapsel eingewogen, mit ∼ 5 mg Zuschlag (V₂O₅ + WO₃) versetzt und bei ∼ 1000°C im Sauerstoffstrom verbrannt. Die dabei entstehenden Verbrennungsgase werden durch eine Absorptionslösung geleitet. Diese setzt sich zusammen aus: ∼ 50 ml Essigsäure ∼ 75 %ig plus 5 ml Hydrazinsulfat-Lösung ∼ 2 %ig. Anschließend wird die Lösung in ein Becherglas überführt und mit Silbernitrat-Maßlösung titriert (dynamische Äquivalenzpunkttitration). Die Einwaage der Probe ist dabei so zu wählen, dass ein Mindestverbrauch von 1 ml Silbernitrat-Maßlösung nicht unterschritten wird.

Die volumetrische Titration ist eine Absolutmethode. Eine Kalibrierung ist daher nicht erforderlich.

Ein Vergleich (Tabelle 1) von erfindungsgemäß hergestellten Polyphenoldisulfiden (Beispiele B1 und B2) mit Polyphenoldisulfiden des Standes der Technik (Vergleichsbeispiele V1 bis V3) zeigt, dass erfindungsgemäß hergestellte Polyphenoldisulfide einen höheren Anteil an dimeren und polymeren Phenoldisulfiden und einen geringeren Anteil an Monosulfideinheiten sowie einen geringeren Gehalt an elementarem Schwefel und kovalent gebundenem Chlor aufweist. Darüber hinaus zeichnen sich erfindungsgemäß hergestellte Polyphenoldisulfide durch eine höhere Temperaturstabilität aus.

### B. Flammschutzmittel und Synergisten

In den Beispielen wurden die Flammschutzmittel FR1 bis FR2 sowie die Synergisten S1 bis S4 eingesetzt.

**Flammschutzmittel:**

| | |
|---|---|
| Triphenylphosphat | FR1 |
| Phosphorsäure-6-(diphenoxy-phosphoryloxy)-hexahydro-furo[3,2-b]-furan-3yl-ester diphenylester | FR2 |

**Synergisten:**

| | |
|---|---|
| Poly-(*tert*.-butylphenoldisulfid) | S1 |
| Poly-(*tert*.-pentylphenoldisulfid) | S2 |
| Poly-(*tert*.-butylphenoldisulfid) | S3 |
| Poly-(*tert*.-pentylphenoldisulfid) | S4 |

Die in den Beispielen eingesetzten Flammschutzmittel und Synergisten wurden kommerziell erworben, nach literaturbekannten Vorschriften oder wie unter Punkt A beschrieben synthetisiert.
- FR1:: Kommerziell erhältlich als Disflamoll® TP von der Firma Lanxess.
- FR2:: Synthetisiert wie in PCT/EP2010/069796 beschrieben.

- S1:: Vultac TB7 der Firma Arkema.
- S2:: Vultac 3 der Firma Arkema.
- S3, S4:: Synthetisiert gemäß Beispiel 6 bzw. 8.

### C. Anwendungsbeispiele

### Beschreibung der Versuche:

Die Ermittlung des Brandverhaltens der Schaumstoffplatten erfolgte, wenn nicht anders angegeben, bei einer Schaumstoffdichte von 15 kg/m³ nach DIN 4102.

In den Vergleichsversuchen wurden die kommerziellen Produkte Vultac TB7 und Vultac 3 (Arkema) eingesetzt.

### Expandierbare Styrolpolymerisate (Extrusionsprozess):

7 Gew.-Teile n-Pentan wurden in Polystyrol 158K (Mw = 261 000 g/mol, Mn = 77 000 g/mol bestimmt mittels GPC, RI-Detektor, PS als Standard) der BASF SE mit einer Viskositätszahl von 98 ml/g eingemischt. Nach Abkühlen der treibmittelhaltigen Schmelze von ursprünglich 260°C auf eine Temperatur von 190°C wurde eine Polystyrolschmelze, welche die in der Tabelle genannten Flammschutzsysteme und optional athermane Verbindungen wie Graphit, Ruß, etc. enthielt, über einen Seitenstromextruder in den Hauptstrom eingemischt.

Die angegebenen Mengen in Gew.-Teilen beziehen sich auf die gesamte Polystyrolmenge (100 Teile).

Das Gemisch aus Polystyrolschmelze, Treibmittel und Flammschutzmittel wurde mit 60 kg/h durch eine Düsenplatte mit 32 Bohrungen (Durchmesser der Düsen: 0,75 mm) gefördert. Mit Hilfe einer druckbeaufschlagten Unterwassergranulierung wurden kompakte Granulate mit enger Größenverteilung hergestellt.

Das Molekulargewicht der Granulate betrug 220 000 g/mol (Mw) bzw. 80 000 g/mol (Mn) (bestimmt mittels GPC, RI-Detektor, PS als Standard).

Durch Einwirkung von strömendem Wasserdampf wurden die Granulate vorgeschäumt und nach 12-stündiger Lagerung durch weitere Behandlung mit Wasserdampf in einer geschlossenen Form zu Schaumstoffblöcken einer Dichte von 15 kg/m³ verschweißt.

Die Schaumstoffblöcke wurden nachfolgend mittels Heißdrahtschnitt mit einem Styropor-Glühdrahtschneidegerät der Firma Charlotte Kaiser, 67071 Ludwigshafen, Leistung: 180 VA, Primär: 230 V, Sekundär: 0 - 12 V, 15 A) bzw. mittels Kaltdrahtschnitt mit einem oszillierenden Kaltdrahtschneidegeräht der Firma Nuova Idro Press LL, Nr. 397 (Baujahr 1993, Via Consolini, 10, 42026 Ciano d'Enza di Canossa, Italien) in die entsprechenden Normprüfkörper, z. B. für die Ermittlung des Brandverhaltens nach DIN 4102, zugeschnitten.

Die Ergebnisse des Zuschnitts sind in den Tabellen 1 und 2 zusammengestellt.

**Tabelle 1: Geruchsentwicklung von erfindungsgemäßer Polymerzusammensetzung beim Heißdrahtschnitt (Beispiele) und von Vergleichsbeispielen**

| **Beispiel** | **Flammschutzsystem** (Gew.-Teile bezogen auf Polystyrol) | **Schaumstoffdichte** [kg/m3] (ISO 845) | **Geruch beim Zuschnitt** |
|---|---|---|---|
| **VB1** | FR1 (1,5) + S1 (3,5) | 14,8 | signifikant / schwefelig |
| **VB2** | FR1 (1,5) + S2 (2,5) | 15,3 | signifikant / schwefelig |
| **VB3** | FR2 (1,0) + S1 (2,5) | 15,4 | signifikant / schwefelig |
| **VB4** | FR2 (1,0) + S2 (2,5) | 15,3 | signifikant / schwefelig |
| **1** | FR1 (1,5) + S3 (3,5) | 15,0 | gering / schwefelig |
| **2** | FR1 (1,5) + S4 (2,5) | 14,8 | gering / schwefelig |
| **3** | FR2 (1,5) + S3 (3,5) | 15,2 | gering / schwefelig |
| **4** | FR2 (1,5) + S4 (2,5) | 15,5 | gering / schwefelig |

**Tabelle 2: Geruchsentwicklung von erfindungsgemäßer Polymerzusammensetzung beim oszillierenden Kaltdrahtschitt (Beispiele) und von Vergleichsbeispielen**

| **Beispiel** | **Flammschutzsystem** (Gew.-Teile bezogen auf Polystyrol) | **Schaumstoffdichte** [kg/m³] (ISO 845) | **Geruch beim Zuschnitt** |
|---|---|---|---|
| **VB1** | FR1 (1,5) + S1 (3,5) | 14,8 | signifikant / schwefelig |
| **VB2** | FR1 (1,5) + S2 (2,5) | 15,3 | signifikant / schwefelig |
| **VB3** | FR2 (1,0) + S1 (2,5) | 15,4 | signifikant / schwefelig |
| **VB4** | FR2 (1,0) + S2 (2,5) | 15,3 | signifikant / schwefelig |
| **5** | FR1 (1,5) + S3 (3,5) | 15,0 | gering / schwefelig |
| **6** | FR1 (1,5) + S4 (2,5) | 14,8 | gering / schwefelig |
| **7** | FR2 (1,0) + S3 (3,5) | 15,2 | gering / schwefelig |
| **8** | FR2 (1,0) + S4 (2,5) | 15,5 | gering / schwefelig |

Die Ermittlung des Brandverhaltens der Schaumstoffplatten erfolgte nach 72-stündiger Lagerung bei einer Schaumstoffdichte von 15 kg/m³ nach DIN 4102.

Die Brandergebnisse sind in Tabelle 3 zusammengestellt.

**Tabelle 3: Brandverhalten von erfindungsgemäßer Polymerzusammensetzung (Beispiele) und von Vergleichsbeispielen**

| **Beispiel** | **Flammschutzsystem** (Gew.-Teile bezogen auf Polystyrol) | **Brandtest (B2 nach DIN 4102) / Verlöschzeit (s)** |
|---|---|---|
| **VB1** | FR1 (1,5) + S1 (3,5) | bestanden / 4 s |
| **VB2** | FR1 (1,5) + S2 (2,5) | bestanden / 6 s |
| **VB3** | FR2 (1,0) + S1 (2,5) | bestanden / 6 s |
| **VB4** | FR2 (1,0) + S2 (2,5) | bestanden / 5 s |
| **5** | FR1 (1,5) + S3 (3,5) | bestanden / 5 s |
| **6** | FR1 (1,5) + S4 (2,5) | bestanden / 4 s |
| **7** | FR2 (1,0) + S3 (3,5) | bestanden / 5 s |
| **8** | FR2 (1,0) + S4 (2,5) | bestanden / 7 s |

### Extrudierte Polystyrol-Schaumstoffplatten:

100 Gew.-Teile Polystyrol 158K (Mw = 261 000 g/mol, Mn = 77 000 g/mol bestimmt mittels GPC, RI-Detektor, PS als Standard) der BASF SE mit einer Viskositätszahl von 98 ml/g, 0,1 Gew.-Teile Talkum als Keimbildner zur Regelung der Zellgröße und die in der Tabelle angegebenen Gew.-Teile an Flammschutzmitteln und Synergisten werden einem Extruder mit einem inneren Schneckendurchmesser von 120 mm kontinuierlich zugeführt. Durch eine in den Extruder angebrachte Einlassöffnung wird gleichzeitig ein Treibmittelgemisch aus 3,25 Gew.-Teilen Ethanol und 3,5 Gew.-Teilen CO₂ kontinuierlich eingedrückt. Das in dem Extruder bei 200 °C gleichmäßig geknetete Gel wird durch eine Beruhigungszone geführt und nach einer Verweilzeit von 15 Minuten mit einer Austrittstemperatur von 105 °C durch eine 300 mm breite und 1,5 mm weite Düse in die Atmosphäre extrudiert. Der Schaum wird durch einen mit dem Extruder verbundenen Formkanal geführt, wobei eine geschäumte Plattenbahn mit einem Querschnitt 650 mm x 50 mm und einer Dichte von 40 g/l entsteht.

Das Molekulargewicht des Polystyrols betrug 240 000 g/mol (Mw) bzw. 70 000 g/mol (Mn) (bestimmt mittels GPC, RI-Detektor, PS als Standard).

Die Extrusionsergebnisse sind in Tabelle 4 zusammengefasst.

**Tabelle 4: Geruchsentwicklung von erfindungsgemäßer Polymerzusammensetzung beim Heißdrahtschnitt (Beispiele) und von Vergleichsbeispielen**

| **Beispiel** | **Flammschutzsystem** (Gew.-Teile bezogen auf Polystyrol) | **Geruch bei Extrusion** (200 °C) | **Geruch der extrudierten Schaumstoffplatten** (Lagerzeit: 48 h bei RT) |
|---|---|---|---|
| **VB5** | FR1 (1,0) + S1 (2,5) | signifikant / schwefelig | signifikant / schwefelig |
| **VB6** | FR1 (1,0) + S2 (2,0) | signifikant / schwefelig | signifikant / schwefelig |
| **VB7** | FR2 (0,75) + S1 (2,5) | signifikant / schwefelig | signifikant / schwefelig |
| **VB8** | FR2 (0,75) + S2 (2,0) | signifikant / schwefelig | signifikant / schwefelig |
| **9** | FR1 (1,0) + S3 (2,5) | sehr gering / schwefelig | geruchsneutral |
| **10** | FR1 (1,0) + S4 (2,0) | sehr gering / schwefelig | geruchsneutral |
| **11** | FR2 (0,75) + S3 (2,5) | sehr gering / schwefelig | geruchsneutral |
| **12** | FR2 (0,75) + S4 (2,0) | sehr gering / schwefelig | geruchsneutral |

Das Produkt wurde anschließend mechanisch in Platten mit einer Dicke von 1 cm geschnitten. Geprüft wurde das Brandverhalten der Proben nach einer Lagerungszeit von 30 Tagen nach DIN 4102.

Die Brandergebnisse sind in Tabelle 5 zusammengefasst.

**Tabelle 5: Brandverhalten von erfindungsgemäßer Polymerzusammensetzung (Beispiele) und von Vergleichsbeispielen**

| **Beispiel** | **Flammschutzsystem** (Gew.-Teile bezogen auf Polystyrol) | **Brandtest (B2 nach DIN 4102) / Verlöschzeit (s)** |
|---|---|---|
| **VB5** | FR1 (1,0) + S1 (2,5) | bestanden/ 12 s |
| **VB6** | FR1 (1,0) + S2 (2,0) | bestanden/ 8 s |
| **VB7** | FR2 (0,75) + S3 (2,5) | bestanden/ 11 s |
| **VB8** | FR2 (0,75) + S4 (2,0) | bestanden/ 12 s |
| **9** | FR1 (1,0) + S3 (2,5) | bestanden / 10 s |
| **10** | FR1 (1,0) + S4 (2,0) | bestanden/ 9 s |
| **11** | FR2 (0,75) + S3 (2,5) | bestanden/ 11 s |
| **12** | FR2 (0,75) + S4 (2,0) | bestanden/ 12 s |

## Patentansprüche

1. Verfahren zur Herstellung eines Polyphenoldisulfids der Formel (I) 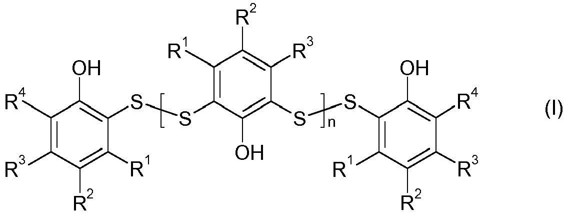 durch Umsetzung von einem oder mehreren Phenolen der Formel (II) 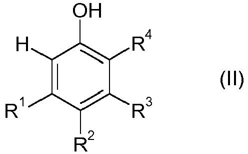 mit S₂Cl₂ (III) in einem Lösungsmittel L,
wobei das Lösungsmittel L die Eigenschaft besitzt, dass
a) es mindestens 80 mol-% der im Laufe der Umsetzung entstehenden HCl komplexiert und/oder neutralisiert, wobei das Lösungsmittel L eine oder mehrere Lewis- und/oder Brønsted-basische Lösungsmittelkomponenten enthält, welche HCl komplexieren und/oder neutralisieren, wobei die einen oder mehreren Lewis- und/oder Brønsted-basischen Lösungsmittelkomponenten Nitrile mit 2-8 C-Atomen pro Cyanogruppe, Carbonsäureamide mit 2 bis 8 C-Atomen pro Carbonsäureamidgruppe, Harnstoffe mit 2 bis 8 C-Atomen pro Harnstoffgruppe, Thioharnstoffe mit 2 bis 8 C-Atomen pro Thioharnstoffgruppe, Sulfoxide mit 2 bis 8 C-Atomen pro Sulfoxygruppe, Amine mit 4 bis 20 C-Atomen pro Aminogruppe sind; oder
b) mindestens 80 mol-% der im Laufe der Umsetzung entstehenden HCl in dem Lösungsmittel L unlöslich ist;
und
wobei die Symbole R¹, R², R³, R⁴ und n folgende Bedeutungen haben:
R¹, R², R³, R⁴ sind gleich oder verschieden C₁-C₁₈-Alkyl, C₂-C₁₈-Alkenyl, C₂-C₁₈-Alkinyl, C₆-C₁₂-Aryl, C₃-C₁₀-Cycloalkyl, C₆-C₁₂-Aryl-C₁-C₁₈-Alkyl, eine Heteroarylgruppe, die ein oder mehrere Heteroatome aus der Gruppe N, O und S enthält, O-(C₁-C₁₈)-Alkyl, O-(C₂-C₁₈)-Alkenyl, O-(C₂-C₁₈)-Alkinyl, O-(C₆-C₁₂)-Aryl, O-(C₃-C₁₀)-Cycloalkyl, (C₆-C₁₂)-Aryl-(C₁-C₁₈)-Alkyl-O, S-(C₁-C₁₈)-Alkyl, S-(C₂-C₁₈)-Alkenyl, S-(C₂-C₁₈)-Alkinyl, S-(C₆-C₁₂)-Aryl, S-(C₃-C₁₀)-Cycloalkyl, (C₆-C₁₂)-Aryl-(C₁-C₁₈)-Alkyl-S, OH, F, Cl, Br oder H;
n ist eine ganze Zahl von 1 bis 1000;
mit der Maßgabe, dass R⁴ in mindestens einem der einen oder mehreren Phenole der Formel (II) H ist.

2. Verfahren gemäß Anspruch 1, wobei n eine ganze Zahl von 3 bis 1000 ist.

3. Verfahren gemäß Anspruch 1, wobei die Symbole R¹, R², R³, R⁴ und n folgende Bedeutungen haben:
R² ist gleich oder verschieden C₁-C₁₆-Alkyl oder C₆-C₁₂-Aryl;
R¹, R³, R⁴ sind gleich oder verschieden H, C₁-C₁₈-Alkyl, C₃-C₁₀-Cycloalkyl oder C₆-C₁₂-Aryl;
n ist eine ganze Zahl von 1 bis 250.

4. Verfahren gemäß einem der Ansprüche 1 bis 3, wobei n eine ganze Zahl von 3 bis 100 ist.

5. Verfahren gemäß einem der Ansprüche 1 bis 4, wobei das Polyphenoldisulfid der Formel (I) Poly-(*tert*.-butylphenoldisulfid), Poly-(*tert*.-pentylphenoldisulfid) oder Poly-(para-phenylphenoldisulfid) ist.

6. Verfahren zur Herstellung eines Polyphenoldisulfids der Formel (I) 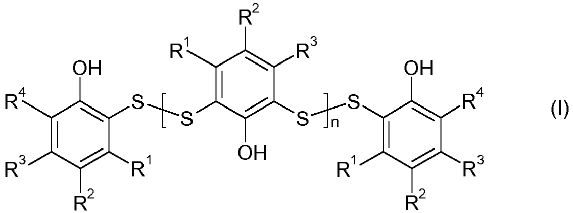 durch Umsetzung von einem oder mehreren Phenolen der Formel (II) 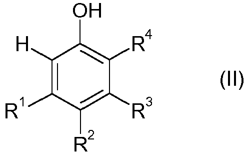 mit S₂Cl₂ (III) in einem Lösungsmittel L,
wobei das Lösungsmittel L die Eigenschaft besitzt, dass es mindestens 80 mol-% der im Laufe der Umsetzung entstehenden HCl komplexiert und/oder neutralisiert, wobei das Lösungsmittel L eine oder mehrere Lewis- und/oder Brønsted-basische Lösungsmittelkomponenten enthält, welche HCl komplexieren und/oder neutralisieren, wobei die einen oder mehreren Lewis- und/oder Brønsted-basischen Lösungsmittelkomponenten Nitrile mit 2-8 C-Atomen pro Cyanogruppe, Carbonsäureamide mit 2 bis 8 C-Atomen pro Carbonsäureamidgruppe, Harnstoffe mit 2 bis 8 C-Atomen pro Harnstoffgruppe, Thioharnstoffe mit 2 bis 8 C-Atomen pro Thioharnstoffgruppe, Sulfoxide mit 2 bis 8 C-Atomen pro Sulfoxygruppe, Amine mit 4 bis 20 C-Atomen pro Aminogruppe sind;
und
wobei die Symbole R¹, R², R³, R⁴ und n folgende Bedeutungen haben:
R² ist gleich oder verschieden C₁-C₁₀-Alkyl oder C₆-C₁₂-Aryl;
R¹, R³, R⁴ sind H;
n ist eine ganze Zahl von 0 bis 150.

7. Verfahren gemäß Anspruch 6, wobei das Polyphenoldisulfid der Formel (I) Di-(*tert*.-butylphenol)disulfid, Di-(*tert*.-pentylphenol)disulfid, Poly-(*tert*.-butylphenoldisulfid), Poly-(*tert*.-pentylphenoldisulfid), Di-(para-phenylphenol)disulfid oder Poly-(para-phenylphenoldisulfid) ist.

8. Verfahren gemäß einem der Ansprüche 1 bis 5, wobei das Lösungsmittel L die Eigenschaft besitzt, dass es mindestens 80 mol-% der im Laufe der Umsetzung entstehenden HCl komplexiert und/oder neutralisiert (a).

9. Verfahren gemäß einem der Ansprüche 1 bis 5, wobei das Lösungsmittel L die Eigenschaft besitzt, dass mindestens 80 mol-% der im Laufe der Umsetzung entstehenden HCl in dem Lösungsmittel L unlöslich ist (b).

10. Verfahren gemäß Anspruch 9, wobei das Lösungsmittel L die Eigenschaft besitzt, dass HCl in dem Lösungsmittel L bei einer Temperatur von 20 °C und einem Druck von 1013 mbar eine Löslichkeit von höchstens 0,05 g HCl/g Lösungsmittel L aufweist.

11. Verfahren gemäß Anspruch 9 oder 10, wobei das Lösungsmittel L folgende Komponenten enthält:
(i) 20 bis 100 Gew.-% (bezogen auf L) einer oder mehrerer Lösungsmittelkomponenten L_{X} gewählt aus der Gruppe bestehend aus linearen oder verzweigten C₅-C₂₀-Alkanen, C₅-C₂₀-Cycloalkanen, Petrolethern und anderen flüssigen Gemischen der genannten gesättigten Kohlenwasserstoffe; und
(ii) 0 bis 80 Gew.-% (bezogen auf L) einer oder mehrerer Lösungsmittelkomponenten L_{Y} gewählt aus der Gruppe bestehend aus Benzol, Toluol, o-Xylol, m-Xylol, p-Xylol, Xylol-Isomerengemische, Ethylbenzol und Cumol;
wobei die Summe der Lösungsmittelkomponenten L_{X} und L_{Y} den Wert 100 Gew.-% (bezogen auf L) nicht übersteigt.

12. Polyphenoldisulfid der Formel (I), erhältlich durch das Verfahren gemäß einem der Ansprüche 1 bis 5 und 8 bis 11.

13. Polyphenoldisulfid der Formel (I), erhältlich durch das Verfahren gemäß Anspruch 6 oder 7, wobei n ≥ 1 ist.

14. Polyphenoldisulfid der Formel (Ib), 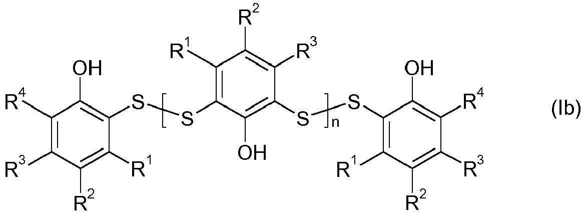 wobei die Symbole R¹, R², R³, R⁴ und n folgende Bedeutungen haben:
R² ist gleich oder verschieden C₆-C₁₂-Aryl;
R¹, R³, R⁴ sind gleich oder verschieden C₆-C₁₂-Aryl oder H;
n ist eine ganze Zahl von 0 bis 1000.

15. Diphenoldisulfid der Formel (la), 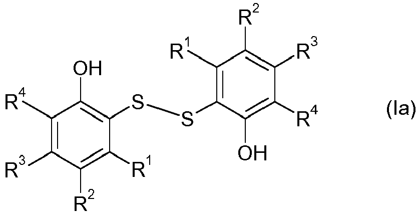 wobei die Symbole R¹, R², R³ und R⁴ folgende Bedeutungen haben:
R² ist gleich oder verschieden C₆-C₁₂-Aryl;
R¹, R³, R⁴ sind gleich oder verschieden C₁-C₁₈-Alkyl, C₂-C₁₈-Alkenyl, C₂-C₁₈-Alkinyl, C₆-C₁₂-Aryl, C₃-C₁₀-Cycloalkyl, C₆-C₁₂-Aryl-C₁-C₁₈-Alkyl, eine Heteroarylgruppe, die ein oder mehrere Heteroatome aus der Gruppe N, O und S enthält, O-(C₁-C₁₈)-Alkyl, O-(C₁-C₁₈)-Alkenyl, O-(C₂-C₁₈)-Alkinyl, O-(C₆-C₁₂)-Aryl, O-(C₃-C₁₀)-Cycloalkyl, (C₆-C₁₂)-Aryl-(C₁-C₁₈)-Alkyl-O, S-(C₁-C₁₈)-Alkyl, S-(C₂-C₁₈)-Alkenyl, S-(C₂-C₁₈)-Alkinyl, S-(C₆-C₁₂)-Aryl, S-(C₃-C₁₀)-Cycloalkyl, (C₆-C₁₂)-Aryl-(C₁-C₁₈)-Alkyl-S, OH, F, Cl, Br oder H.

16. Diphenoldisulfid der Formel

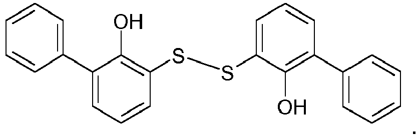

17. Flammschutzsystem, enthaltend
i) mindestens ein Polyphenoldisulfid der Formel (I) erhältlich durch das Verfahren gemäß einem der Ansprüche 1 bis 11; und
ii) mindestens eine halogenfreie organische Phosphorverbindung mit einem Phosphorgehalt im Bereich von 0,5 bis 40 Gew.-%, bezogen auf die Phosphorverbindung.

18. Polymerzusammensetzung, enthaltend ein oder mehrere Polymere und das Flammschutzsystem gemäß Anspruch 17.

## Claims

1. A process for preparing a polyphenol disulfide of formula (I) 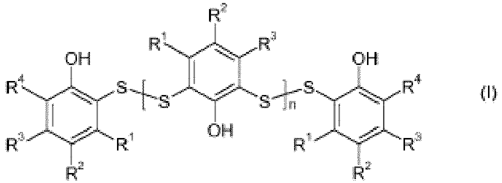 by reacting one or more phenols of formula (II) 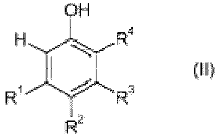 with S₂Cl₂ (III) in a solvent L,
a property possessed by the solvent L being that
a) it complexes and/or neutralizes at least 80 mol% of the HCl forming in the course of the reaction, the solvent L comprising one or more Lewis- and/or Brønsted-basic solvent components which complex and/or neutralize HCl, the one or more Lewis- and/or Brønsted-basic solvent components being nitriles having 2-8 carbon atoms per cyano group, carboxamides having 2 to 8 carbon atoms per carboxamide group, ureas having 2 to 8 carbon atoms per urea group, thioureas having 2 to 8 carbon atoms per thiourea group, sulfoxides having 2 to 8 carbon atoms per sulfoxy group, amines having 4 to 20 carbon atoms per amino group; or
b) at least 80 mol% of the HCl forming in the course of the reaction is insoluble in the solvent L;
and
where the symbols R¹, R², R³, R⁴ and n have the following meanings:
R¹, R², R³ and R⁴ are C₁-C₁₈-alkyl, C₂-C₁₈-alkenyl, C₂-C₁₈-alkynyl, C₆-C₁₂-aryl, C₃-C₁₀-cycloalkyl, C₆-C₁₂-aryl-C₁-C₁₈-alkyl, a hetaryl group comprising one more heteroatoms from the group N, O and S, O-(C₁-C₁₈)-alkyl, O-(C₂-C₁₈)-alkenyl, O-(C₂-C₁₈)-alkynyl, O-(C₆-C₁₂)-aryl, O-(C₃-C₁₀)-cycloalkyl, (C₆-C₁₂)-aryl-(C₁-C₁₈)-alkyl-O, S-(C₁-C₁₈)-alkyl, S-(C₂-C₁₈)-alkenyl, S-(C₂-C₁₈)-alkynyl, S-(C₆-C₁₂)-aryl, S-(C₃-C₁₀)-cycloalkyl, (C₆-C₁₂)-aryl-(C₁-C₁₈)-alkyl-S, OH, F, Cl, Br or H and the same or different in each occurrence;
n is an integer from 1 to 1000;
with the proviso that R⁴ is H in at least one of the one or more phenols of formula (II).

2. The process according to claim 1 wherein n is an integer from 3 to 1000.

3. The process according to claim 1 wherein the symbols R¹, R², R³, R⁴ and n have the following meanings:
R² is C₁-C₁₆-alkyl or C₆-C₁₂-aryl and the same or different in each occurrence;
R¹, R³ and R⁴ are H, C₁-C₁₈-alkyl, C₃-C₁₀-cycloalkyl or C₆-C₁₂-aryl and the same or different in each occurrence;
n is an integer from 1 to 250.

4. The process according to any of claims 1 to 3 wherein n is an integer from 3 to 100.

5. The process according to any of claims 1 to 4 wherein the polyphenol disulfide of formula (I) is poly(*tert*-butylphenol disulfide), poly(*tert-*pentylphenol disulfide) or poly(para-phenylphenol disulfide).

6. A process for preparing a polyphenol disulfide of formula (I) 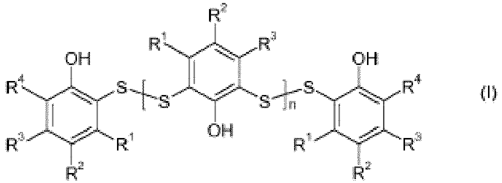 by reacting one or more phenols of formula (II) 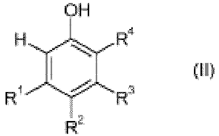 with S₂Cl₂ (III) in a solvent L,
a property possessed by the solvent L being that it complexes and/or neutralizes at least 80 mol% of the HCl forming in the course of the reaction, the solvent L comprising one or more Lewis- and/or Brønsted-basic solvent components which complex and/or neutralize HCl, the one or more Lewis- and/or Brønsted-basic solvent components being nitriles having 2-8 carbon atoms per cyano group, carboxamides having 2 to 8 carbon atoms per carboxamide group, ureas having 2 to 8 carbon atoms per urea group, thioureas having 2 to 8 carbon atoms per thiourea group, sulfoxides having 2 to 8 carbon atoms per sulfoxy group, amines having 4 to 20 carbon atoms per amino group;
and
where the symbols R¹, R², R³, R⁴ and n have the following meanings:
R² is C₁-C₁₀-alkyl or C₆-C₁₂-aryl and the same or different in each occurrence;
R¹, R³ and R⁴ are H;
n is an integer from 0 to 150.

7. The process according to claim 6 wherein the polyphenol disulfide of formula (I) is di(tertbutylphenol) disulfide, di(tert-pentylphenol) disulfide, poly(tert-butylphenol disulfide), poly(tert-pentylphenol disulfide), di(para-phenylphenol) disulfide or poly(para-phenylphenol disulfide).

8. The process according to any of claims 1 to 5 wherein a property possessed by the solvent L is that it complexes and/or neutralizes at least 80 mol% of the HCl forming in the course of the reaction (a).

9. The process according to any of claims 1 to 5 wherein a property possessed by the solvent L is that at least 80 mol% of the HCl forming in the course of the reaction is insoluble in the solvent L (b).

10. The process according to claim 9 wherein a property possessed by the solvent L is that HCl has a solubility of at most 0.05 g HCl/g solvent L in the solvent L at a temperature of 20°C and a pressure of 1013 mbar.

11. The process according to claim 9 or 10 wherein the solvent L comprises the following components:
(i) 20 to 100 wt% (based on L) of one or more solvent components L_{X} selected from the group consisting of linear or branched C₅-C₂₀-alkanes, C₅-C₂₀-cycloalkanes, petroleum ethers and other liquid mixtures of the stated saturated hydrocarbons; and
(ii) 0 to 80 wt% (based on L) of one or more solvent components L_{Y} selected from the group consisting of benzene, toluene, o-xylene, m-xylene, p-xylene, xylene isomer mixtures, ethylbenzene and cumene;
where the sum of the solvent components L_{X} and L_{Y} does not exceed a value of 100 wt% (based on L).

12. A polyphenol disulfide of formula (I) obtainable by the process according to any of claims 1 to 5 and 8 to 11.

13. A polyphenol disulfide of formula (I) obtainable by the process according to claim 6 or 7 wherein n ≥ 1.

14. A polyphenol disulfide of formula (Ib), 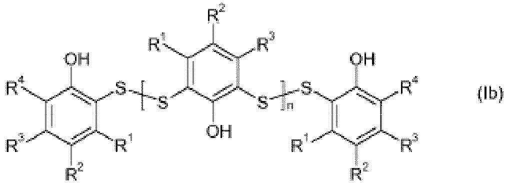 where the symbols R¹, R², R³, R⁴ and n have the following meanings:
R² is C₆-C₁₂-aryl and the same or different in each occurrence;
R¹, R³ and R⁴ are C₆-C₁₂-aryl or H and the same or different in each occurrence;
n is an integer from 0 to 1000.

15. A diphenol disulfide of formula (Ia) 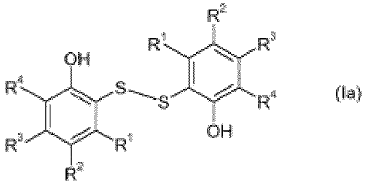 where the symbols R¹, R², R³ and R⁴ have the following meanings:
R² is C₆-C₁₂-aryl and the same or different in each occurrence;
R¹, R³ and R⁴ are C₁-C₁₈-alkyl, C₂-C₁₈-alkenyl, C₂-C₁₈-alkynyl, C₆-C₁₂-aryl, C₃-C₁₀-cycloalkyl, C₆-C₁₂-aryl-C₁-C₁₈-alkyl, a hetaryl group comprising one more hetero atoms from the group N, O and S, O-C₁-C₁₈)-alkyl, O-(C₂-C₁₈)-alkenyl, O-(C₂-C₁₈)-alkynyl, O-(C₆-C₁₂)-aryl, O-(C₃-C₁₀)-cycloalkyl, (C₆-C₁₂)-aryl-(C₁-C₁₈)-alkyl-O, S-(C₁-C₁₈)-alkyl, S-(C₂-C₁₈)-alkenyl, S-(C₂-C₁₈)-alkynyl, S-(C₆-C₁₂)-aryl, S-(C₃-C₁₀)-cycloalkyl, (C₆-C₁₂)-aryl-(C₁-C₁₈)-alkyl-S, OH, F, Cl, Br or H and the same or different in each occurrence.

16. The diphenol disulfide of formula

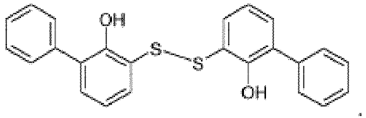

17. A flame retardant system comprising
i) at least one polyphenol disulfide of formula (I) obtainable by the process according to any of claims 1 to 11; and
ii) at least one halogen-free organophosphorus compound having a phosphorus content in the range from 0.5 to 40 wt%, based on the phosphorus compound.

18. A polymer composition comprising one or more polymers and the flame retardant system according to claim 17.

## Revendications

1. Procédé pour la préparation d'un poly(disulfure de phénol) de formule (I) 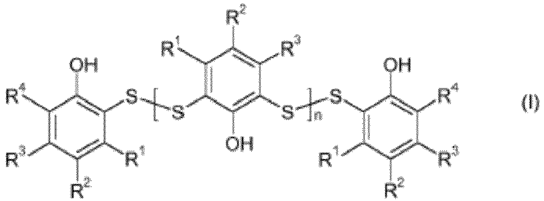 par transformation d'un ou de plusieurs phénols de formule (II), 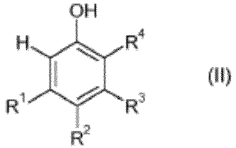 avec du S₂Cl₂ (III) dans un solvant L,
le solvant L possédant la propriété selon laquelle
a) il complexe et/ou neutralise au moins 80% en mole du HCl se formant au cours de la transformation, le solvant L contenant un ou plusieurs constituants de solvant basiques de Lewis et/ou de Brønsted, qui complexent et/ou neutralisent le HCl, ledit un ou lesdits plusieurs constituants de solvant basiques de Lewis et/ou de Brønsted étant des nitriles comprenant 2-8 atomes de carbone par groupe cyano, des amides d'acide carboxylique comprenant 2 à 8 atomes de carbone par groupe amide d'acide carboxylique, des urées comprenant 2 à 8 atomes de carbone par groupe urée, des thio-urées comprenant 2 à 8 atomes de carbone par groupe thio-urée, des sulfoxydes comprenant 2 à 8 atomes de carbone par groupe sulfoxyde, des amines comprenant 4 à 20 atomes de carbone par groupe amino ; ou
b) au moins 80% en mole du HCl se formant au cours de la transformation sont insolubles dans le solvant L ;
et
les symboles R¹, R², R³, R⁴ et n présentant les significations suivantes :
R¹, R², R³, R⁴ sont identiques ou différents et représentent C₁-C₁₈-alkyle, C₂-C₁₈-alcényle, C₂-C₁₈-alcynyle, C₆-C₁₂-aryle, C₃-C₁₀-cycloalkyle, C₆-C₁₂-aryl-C₁-C₁₈-alkyle, un groupe hétéroaryle, qui contient un ou plusieurs hétéroatomes du groupe constitué par N, O et S, O-(C₁-C₁₈)-alkyle, O-(C₂-C₁₈)-alcényle, O-(C₂-C₁₈)-alcynyle, O-(C₆-C₁₂)-aryle, O-(C₃-C₁₀)-cycloalkyle, (C₆-C₁₂)-aryl- (C₁-C₁₈)-alkyl-O, S-(C₁-C₁₈)-alkyle, S-(C₂-C₁₈)-alcényle, S-(C₂-C₁₈)-alcynyle, S-(C₆-C₁₂)-aryle, S-(C₃-C₁₀)-cycloalkyle, (C₆-C₁₂)-aryl-(C₁-C₁₈)-alkyl-S, OH, F, Cl, Br ou H ;
n est un nombre entier de 1 à 1000 ;
à condition que R⁴ dans au moins un dudit un ou desdits plusieurs phénols de formule (II) représente H.

2. Procédé selon la revendication 1, n étant un nombre entier de 3 à 1000.

3. Procédé selon la revendication 1, les symboles R¹,
R², R³, R⁴ et n présentant les significations suivantes :
R² est identique ou différent et signifie C₁-C₁₆-alkyle ou C₆-C₁₂-aryle ;
R¹, R³, R⁴ sont identiques ou différents et signifient H, C₁-C₁₈-alkyle, C₃-C₁₀-cycloalkyle ou C₆-C₁₂-aryle ;
n représente un nombre entier de 1 à 250.

4. Procédé selon l'une quelconque des revendications 1 à 3, n représentant un nombre entier de 3 à 100.

5. Procédé selon l'une quelconque des revendications 1 à 4, le poly(disulfure de phénol) de formule (I) étant le poly-(disulfure de tert-butylphénol), le poly-(disulfure de tert-pentylphénol) ou le poly-(disulfure de para-phénylphénol).

6. Procédé pour la préparation d'un poly(disulfure de phénol) de formule 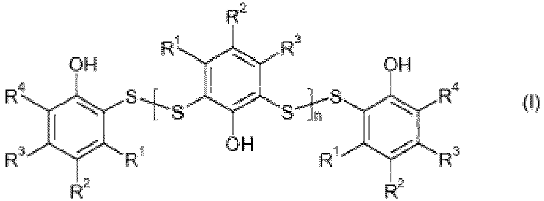 par transformation d'un ou de plusieurs phénols de formule (II), 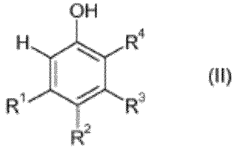 avec du S₂Cl₂ (III) dans un solvant L,
le solvant L possédant la propriété selon laquelle il complexe et/ou neutralise au moins 80% en mole du HCl se formant au cours de la transformation, le solvant L contenant un ou plusieurs constituants de solvant basiques de Lewis et/ou de Brønsted, qui complexent et/ou neutralisent le HCl, ledit un ou lesdits plusieurs constituants de solvant basiques de Lewis et/ou de Brønsted étant des nitriles comprenant 2-8 atomes de carbone par groupe cyano, des amides d'acide carboxylique comprenant 2 à 8 atomes de carbone par groupe amide d'acide carboxylique, des urées comprenant 2 à 8 atomes de carbone par groupe urée, des thio-urées comprenant 2 à 8 atomes de carbone par groupe thio-urée, des sulfoxydes comprenant 2 à 8 atomes de carbone par groupe sulfoxyde, des amines comprenant 4 à 20 atomes de carbone par groupe amino ;
et
les symboles R¹, R², R³, R⁴ et n présentant les significations suivantes :
R² est identique ou différent et signifie C₁-C₁₀-alkyle ou C₆-C₁₂-aryle ;
R¹, R³, R⁴ représentent H ;
n représente un nombre entier de 0 à 150.

7. Procédé selon la revendication 6, le poly(disulfure de phénol) de formule (I) étant le disulfure de di(tert-butylphénol), le disulfure de di(tert-pentylphénol), le poly-(disulfure de tert-butylphénol), le poly-(disulfure de tert-pentylphénol), le disulfure de di-(para-phénylphénol) ou le poly-(disulfure de para-phénylphénol).

8. Procédé selon l'une quelconque des revendications 1 à 5, le solvant L possédant la propriété selon laquelle il complexe et/ou neutralise au moins 80% en mole du HCl se formant au cours de la transformation (a).

9. Procédé selon l'une quelconque des revendications 1 à 5, le solvant L possédant la propriété selon laquelle au moins 80% en mole du HCl se formant au cours de la transformation sont insolubles dans le solvant L (b).

10. Procédé selon la revendication 9, le solvant L possédant la propriété selon laquelle le HCl présente, dans le solvant L, à une température de 20°C et à une pression de 1013 mbars, une solubilité d'au plus 0,05 g de HCl/g de solvant L.

11. Procédé selon la revendication 9 ou 10, le solvant L contenant les constituants suivants :
(i) 20 à 100% en poids (par rapport à L) d'un ou de plusieurs constituants de solvant Lₓ choisi(s) dans le groupe constitué par les C₅-C₂₀-alcanes linéaires ou ramifiés, les C₅-C₂₀-cycloalcanes, les éthers de pétrole et d'autres mélanges liquides des hydrocarbures saturés mentionnés ; et
(ii) 0 à 80% en poids (par rapport à L) d'un ou de plusieurs constituants de solvant L_{y} choisis dans le groupe constitué par le benzène, le toluène, l'o-xylène, le m-xylène, le p-xylène, les mélanges d'isomères de xylène, l'éthylbenzène et le cumène ;
la somme des constituants de solvant Lₓ et L_{y} ne dépassant pas la valeur de 100% en poids (par rapport à L).

12. Poly(disulfure de phénol) de formule (I), pouvant être obtenu par le procédé selon l'une quelconque des revendications 1 à 5 et 8 à 11.

13. Poly(disulfure de phénol) de formule (I), pouvant être obtenu par le procédé selon la revendication 6 ou 7, n étant ≥ 1.

14. Poly(disulfure de phénol) de formule (Ib), 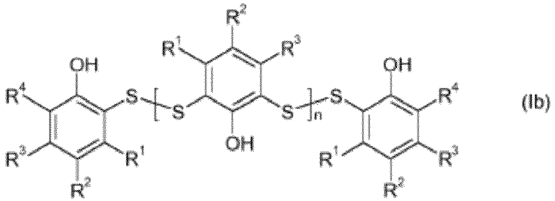 les symboles R¹, R², R³, R⁴ et n présentant les significations suivantes :
R² est identique ou différent et signifie C₆-C₁₂-aryle ;
R¹, R³, R⁴ sont identiques ou différents et représentent C₆-C₁₂-aryle ou H ;
n représente un nombre entier de 0 à 1000.

15. Disulfure de diphénol de formule (Ia), 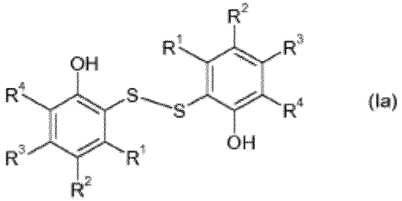 les symboles R¹, R², R³ et R⁴ présentant les significations suivantes :
R² est identique ou différent et signifie C₆-C₁₂-aryle ;
R¹, R³, R⁴ sont identiques ou différents et représentent C₁-C₁₈-alkyle, C₂-C₁₈-alcényle, C₂-C₁₈-alcynyle, C₆-C₁₂-aryle, C₃-C₁₀-cycloalkyle, C₆-C₁₂-aryl-C₁-C₁₈-alkyle, un groupe hétéroaryle, qui contient un ou plusieurs hétéroatomes du groupe constitué par N, O et S, O-(C₁-C₁₈)-alkyle, O-(C₂-C₁₈)-alcényle, O-(C₂-C₁₈)-alcynyle, O-(C₆-C₁₂)-aryle, O-(C₃-C₁₀)-cycloalkyle, (C₆-C₁₂)-aryl-(C₁-C₁₈)-alkyl-O, S-(C₁-C₁₈)-alkyle, S-(C₂-C₁₈)-alcényle, S-(C₂-C₁₈)-alcynyle, S-(C₆-C₁₂)-aryle, S-(C₃-C₁₀)-cycloalkyle, (C₆-C₁₂)-aryl-(C₁-C₁₈)-alkyl-S, OH, F, Cl, Br ou H.

16. Disulfure de diphénol de formule

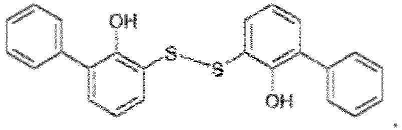

17. Système d'agent ignifuge contenant
i) au moins un poly(disulfure de phénol) de formule (I), pouvant être obtenu par le procédé selon l'une quelconque des revendications 1 à 11 ; et
ii) au moins un composé phosphoré organique exempt d'halogène, présentant une teneur en phosphore dans la plage de 0,5 à 40% en poids, par rapport au composé phosphoré.

18. Composition polymère, contenant un ou plusieurs polymères et le système d'agent ignifuge selon la revendication 17.
